(19) Europäisches Patentamt
European Patent Office
Office européen des brevets

(11) Publication number: **0 188 864 B1**

# (12) EUROPEAN PATENT SPECIFICATION

(45) Date of publication of patent specification: **11.12.91**

(51) Int. Cl.5: **C12N 15/25**, C07H 21/04, C12P 21/02, C07K 13/00

(21) Application number: **85303701.8**

(22) Date of filing: **24.05.85**

## (54) DNA encoding human interleukin 1 alpha and the amino acid chain corresponding thereto and vectors and hosts containing such DNA; and the preparatiion thereof.

(30) Priority: **17.01.85 US 692416**
**10.04.85 US 721765**

(43) Date of publication of application:
**30.07.86 Bulletin 86/31**

(45) Publication of the grant of the patent:
**11.12.91 Bulletin 91/50**

(84) Designated Contracting States:
**AT BE CH DE FR GB IT LI LU NL SE**

(56) References cited:
**EP-A- 0 165 654**
**EP-A- 0 188 920**

**FEDERATION PROCEEDINGS, vol. 42, no. 9, June 1983, (Washington DC) L.B. LACHMAN : "Human interleukin 1: purification and properties" pages 2639-2645**

**NATURE, vol. 302, March 24, 1983 (New York, London) T. TANIGUCHI et al. "Structure and expression of a cloned cDNA for human interleukin-2" pages 305-310**

(73) Proprietor: **IMMUNEX CORPORATION**
**Immunex Building 51 University Street**
**Seattle, WA 98101(US)**

(72) Inventor: **Cerretti, Douglas P.**
**2415 West Bertona**
**Seattle Washington 98199(US)**
Inventor: **Conlon III, Paul J.**
**4805 Stanford Avenue N.E.**
**Seattle Washington 98105(US)**
Inventor: **Cosman, David J.**
**310-12th Avenue East**
**Seattle Washington 98102(US)**
Inventor: **Grabstein, Kenneth H.**
**5829 N.E. 75th Street 443**
**Seattle Washington 98115(US)**
Inventor: **Hopp, Thomas P.**
**4842-51st S.W.**
**Seattle Washington 98116(US)**
Inventor: **Kronheim, Shirley R.**
**11526-36th N.E.**
**Seattle Washington 98125(US)**

EP 0 188 864 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Proc.Nat.Acad.Sci. 81 (1984), pp. 7907-7911

Nature 312 (1984), pp. 458-462

Nature 309 (1984), pp. 56-59

J. Immunol. 132 (1984), pp. 1317-1322

J. Immunol. 131 (1983), pp. 1834-1837

Inventor: **Larsen, Alf D.**
**320 Summit Avenue East 15**
**Seattle Washington 98102(US)**
Inventor: **March, Carl J.**
**8133 Eighth S.W.**
**Seattle Washington 98106(US)**
Inventor: **Mosley, Bruce A.**
**6833-36th Avenue N.E.**
**Seattle Washington 98115(US)**
Inventor: **Price, Virginia L.**
**2617 Boyer Avenue East**
**Seattle Washington 98102(US)**

(74) Representative: **Sheard, Andrew Gregory et al**
**Kilburn & Strode 30, John Street**
**London WC1N 2DD(GB)**

## Description

The present invention relates to interleukin 1 (hereinafter "IL-1"), and more particularly to the cloning of a gene for human IL-1 by use of hybrid selection procedures and by use of radiolabeled natural and synthetic oligonucleotide probe derived from clones composing sections of the IL-1 gene to screen a complimentary deoxyribonucleic acid ("cDNA") library synthesized from IL-1 messenger ribonucleic acid ("mRNA") and to the characterisation of the screened IL-1 gene. The IL-1 molecule to which the invention relates is now known as IL-1$\alpha$.

IL-1, formerly known in the literature as "lymphocyte activating factor" or "LAF," is a hormone secreted by macrophages while undergoing an immune response. This protein factor regulates a wide range of immunological and nonimmunological responses. For instance, it is considered that IL-1 mediates activities referred to as endogenous or leukocytic pyrogen, B-cell activating factor (BAF), epidermal cell thymocyte activating factor (ETAF), leukocyte endogenous mediator (LEM), bone resorption factor active in rheumatoid arthritis, and a variety of other activities.

Although researchers have identified many of the biological properties of IL-1, the chemical nature of this hormone is not well understood. To date, this has been hampered, at least in part, by the unavailability of sufficient quantities of IL-1 in purified form to carry out necessary investigations.

Attempts have been made in the past to purify and partially characterize IL-1 derived from both human and murine sources. For instance, Mizel, 122 J. Immunol. 2167-2172 (1979), reported the production of murine IL-1 from the macrophage cell line, P388$D_1$, cultured in a supplemented growth medium together with phorbol myristic acetate as an activating agent. The IL-1 from the culture fluid was subjected to ammonium sulfate precipitation, diethyl amino ethyl ("DEAE") cellulose column chromatography, ultrafiltration and Sephacryl S200 column chromatography. The resulting active fractions were analyzed by sodium dodecyl sulfate ("SDS")-polyacrylamide gel electrophoresis ("PAGE") and were found to have a molecular weight in the range of 12,000 to 16,000 daltons. Through isoelectrofocusing ("IEF") in polyacrylamide gels, the pl of the IL-1 was found to be in the range of from 5.0 to 5.4.

In a subsequent communication Mizel et al., 126 J. Immunol. 834-837 (1981), discussed purifying IL-1 from the same P388$D_1$ cell line as used in Mizel, supra, to "apparent homogeneity" by ammonium sulfate precipitation, phenyl Sepharose chromatography, Ultrogel AcA54 gel filtration chromatography and preparative flat-bed IEF. From the IEF the pl of the IL-1 was measured to be about 4.9 to 5.1. Through gel electrophoresis the molecular weight of the IL-1 molecule was determined to be approximately 14,000 daltons.

Researchers have also investigated IL-1 produced from human peripheral blood leukocytes and monocytes. Blyden et al., 118 J. Immunol. 1631-1638 (1977), disclosed a protocol for concentrating IL-1 prepared from human peripheral blood leukocytes by Sephadex G-100 column chromatography. This procedure was reported to result in a four-to-five fold concentration of the crude IL-1. DEAE-Bio-Gel A an ion exchange chromatography was employed to remove the albumin from the serum used during the preparation of the crude IL-1. Next, the collected active fractions were adsorbed onto a hydroxyapatite column. Fractions containing peak IL-1 activity were then applied to a CM-Bio-Gel A cationic exchange resin. By these procedures, about 20% of the initial IL-1 was recovered. The resulting IL-1 was found to have a molecular weight of about 13,000 daltons and a pl of approximately 6.8 to 7.2.

Crude IL-1 prepared from human leukocytes by Togawa et al., 122 J. Immunol. 2112-2118 (1979) was initially processed by membrane filtration and then applied to a Bio-Gel P-100 chromatography column which disclosed two major peaks of activity, one in the range of from 12,000 to 22,000 daltons and another in the range of about 50,000 to 70,000 daltons. Active fractions in the lower molecular weight region of the Bio-Gel P-100 column were pooled, applied to a Blue Sepharose column, and then applied to a DEAE-cellulose ion-exchange chromatography column. Thereafter, the IL-1 containing fractions were pooled and applied to a hydroxylapatite chromatography column. Togawa et al. discovered that when the lower molecular weight IL-1 activity resulting from each of these procedures was reconstituted with 2% human serum, concentrated and rechromatographed on Bio-Gel P-100, a significant portion of the higher molecular weight activity appeared.

In a more recent study, Lachman, 42 Federation Proceedings 2639-2645 (1983), reported preparing IL-1 by culturing peripheral blood monocytes or leukemic cells obtained from acute monocytic leukemia or acute myelomonocytic leukemia patients in a serum supplemented culture medium together with lipopolysaccharide ("LPS") to stimulate IL-1 production. Hollow fiber diafiltration and ultrafiltration were used to separate a lower molecular weight activity from most of the serum proteins. This lower weight activity was subjected to IEF in an Ampholine and sucrose gradient. From this procedure, the IL-1 activity was found to have a pl of about 6.8 to 7.2. The isoelectrofocused IL-1 activity was then subjected to SDS-PAGE which indicated that the human IL-1 being analyzed had a molecular weight of about 11,000 daltons. Lachman reported that the overall recovery of IL-1 activity from the above procedures was poor, in the range of about 4%.

The availability of adequate quantities of homogeneous human IL-1 could be valuable in investigations and possible treatment of autoimmune disorders such as arthritis and lupus erythematosis. Also, human IL-1 in greater purity and larger quantities than heretofore available, could prove useful in achieving successful wound and burn healing.

One potential method of providing relatively large quantities of homogeneous human IL-1 is through recombinant DNA techniques. Recombinant DNA techniques have been developed for economically producing a desired protein once the gene coding for the protein has been isolated and identified. A general discussion of such recombinant DNA techniques for protein production is set forth in the editorial and supporting papers in Vol. 196 of Science (April 1977). However, to take advantage of the recombinant DNA techniques discussed in this reference, the gene coding for human IL-1 must first be isolated.

In accordance with the present invention, a gene coding for human IL-1 was isolated in several sections from a cDNA library with various hybridization techniques. Total human RNA was extracted from cells thought to produce relatively high levels of IL-1. Polyadenylated mRNA was isolated from the total RNA extract. A cDNA library was constructed by reverse transcription of size separated polyadenylated mRNA with reverse transcriptase. The DNA was rendered double-stranded with DNA polymerase I and inserted into an appropriate cloning vector. Resultant recombinant cloning vectors were used to transform an appropriate host.

Transformed hosts were identified and grouped into pools. Plasmid DNA prepared from these pools was hybridized with total RNA, which when translated in vitro gave rise to IL-1 biological activity (IL-1 active mRNA). The pool(s) of clones that hybridized with IL-1 active mRNA was identified and then the putative pool subdivided and the hybrid selection screen repeated. By this procedure, a single transformant was eventually identified. Plasmid DNA was prepared from this transformant and sequenced to establish its nucleotide and amino acid composition. This first clone was radiolabeled and then employed as a hybridization probe to rescreen the entire cDNA library. Plasmid DNA prepared from the transformed hosts was hybridized with the radiolabeled first clone. Pools that gave a positive signal were plated out and rescreened with the probe. Through this process, a single positive colony was identified. Plasmid DNA was prepared from the colony and sequenced. This second clone was found to overlap a portion of the first clone and compose a further section of the IL-1 gene.

The cDNA library was also screened with a synthetic oligonucleotide probe corresponding to a portion of the first clone. The screening procedure was essentially the same as used above with a probe composed of the entire first clone. Through this third screening process, two additional clones (third and fourth) were discovered which overlap portions of the first and second clones. These clones were then employed as probes to further screen the cDNA library and isolate further clones composing portions of the IL-1 gene.

Due to the overlapping nature of the clones that were isolated, the nucleotide and amino acid sequences of the entire open reading frame and the nucleotide sequences of the 5' flanking region and a large 3' flanking region of the IL-1 gene were determined. Also, the entire open reading frame of the IL-1 gene and the coding region of the gene were cloned in mammilian and bacteria host systems, respectively, to express mature Il-1. Thereafter biological assays were conducted to confirm that the expressed protein product was IL-1

According to a first aspect, the present invention provides isolated DNA encoding mature human IL-1α wherein said human IL-1α has the amino acid sequence extending from residue no. 113 to residue no. 271 inclusive in Figures 1A and 1B. The DNA may have the nucleic acid sequence extending from nucleic acid base no. 337 to nucleic acid base no. 813 inclusive in Figures 1A and 1B

The invention also provides recombinant DNA cloning and expression vectors comprising DNA as described above and a host transformed with such a vector. The expression vector may be capable of inducing expression of the DNA in a mammalian or bacterial host. Associated processes for producing a transformant by means of an expression vector and for preparing human IL-1α by culturing such a transformant are also provided.

The invention further provides isolated mature human IL-1α, wherein said human IL-1α has the amino acid sequence extending from residue no. 113 to residue no. 271 inclusive in Figures 1A and 1B, as well as such human IL-1α for use in medicine. Pharmaceutical compositions comprising such human IL-1α, particularly for use in the regulation of immunological responses, in the treatment of autoimmune disorders and in the treatment of arthritis or wound or burn healing, are also provided. Human IL-1α having the sequence set out can be used in the preparation of medicaments for use in such indications.

The details of typical embodiments of the present invention will be described in connection with the accompanyng drawings, in which:

FIGURE 1 (composed of 3 sheets) illustrates the nucleotide sequence and the corresponding amino acid sequence of the IL-1 gene together with a 5' flanking region and an extensive 3' flanking region, with the nucleotides being numbered in each direction from the beginning of the open reading frame of the IL-1 gene, and the corresponding amino acids being numbered from this same location, i.e., the Met residue, as marked with a star, to the termination codon TAG at residue number 271.

FIGURE 2 schematically illustrates the locations of the various clones isolated by the present invention along the nucleotide sequence shown in FIGURE 1 and the overlapping nature of the clones. The boxed portion represents the open reading frame of the gene, with the striped section of the boxed portion indicating the coding region of the mature IL-1 protein. The scale is graduated in base pairs ("bp") x $10^2$.

FIGURE 3 illustrates the strategy employed to clone the entire open reading frame of the IL-1 gene into an SV40 based expression vector used to transform mammalian cells to express functional IL-1.

FIGURE 4 illustrates the strategy employed to clone the coding region of the IL-1 gene into a plasmid used to transform bacterial hosts to express functional IL-1.

## Sources of Human IL-1 Producing Cells

Preferably, a cDNA library, from which genes coding for human IL-1 will be sought, is constructed from cells previously found to produce relatively high levels of other lymphokines, under the assumption that they might also produce human IL-1. These sources may include activated human peripheral blood adherent mononuclear cells. For use in the present invention, the peripheral blood mononuclear cells can be separated from whole blood by standard techniques, such as by Ficoll-Hypaque centrifugation. The leukocytes removed from the blood are cultured *in vitro* in a culture medium containing an appropriate stimulating agent to induce IL-1 secretion.

Rather than being obtained from leukocytes removed from whole blood, IL-1 can alternatively be prepared from monocytes derived from any monocyte rich source. Such monocyte sources include monocytic leukemic spleen cells, lymph cells and alevolar macrophages.

The medium used to culture the peripheral blood leukocytes may consist of commercially available media, such as Eagle's Minimum Essential Medium ("MEM") or Roswell Park Memorial Institute ("RPMI") medium. Additives, which may be individually or in combination added to the culture medium, include glutamine, HEPES buffer and various antibiotics, such as gentamycin, penicillin and streptomycin. In the past, serum also has been commonly used as an additive.

Preferable stimulating agents used in conjunction with the present invention include *Staphylococcus aureus* ("*S. aureus*") or LPS extracted from *Escherichia coli* ("*E. coli*"). In addition, phorbol esters, such as phorbal myristrate 13-acetate, may be employed as a stimulating agent.

The process of culturing the leukocytes to induce secretion of IL-1 may be carried out in various environmental conditions. Preferably, however, the cultures are maintained in the temperature range of approximately 35-38° C in a humidified atmosphere of approximately 5-10% $CO_2$ in air. The quantity of IL-1 released by stimulation of peripheral blood leukocytes with an activating agent varies with time. Applicants have found that optimum levels of IL-1 expression are reached at approximately 24 hours after stimulation.

## Preparation of RNA from Human IL-1 Producing Cells

Total RNA from human potentially IL-1-producing cells is extracted by standard methods, such as disclosed by Chirgwin et al., 18 *Biochemistry* 5294 (1979), and Maniatis et al., *Molecular Cloning, a Laboratory Manual*, Cold Spring Harbor Laboratory, Cold Spring Harbor, New York (1982).

As is well known, when extracting RNA from cells, it is important to minimize ribonuclease ("RNase") activity during the initial stages of extraction. One manner in which this is accomplished is to denature the cellular protein, including the RNase, at a rate that exceeds the rate of RNA hydrolysis by RNase. In the procedures of Chirgwin et al., *supra*, and Maniatis et al., *supra* at 196, this is carried out by use of guanidinium thiocyanate, together with a reducing agent, such as 2-mercaptoethanol (to break up the protein disulfide bonds). The RNA is isolated from the protein by standard techniques, such as phenol/chloroform extraction, ethanol precipitation or sedimentation through cesium chloride. Alternatively, the RNA can be separated from the protein by extraction with guanidine hydrochloride followed by extraction with phenol/chloroform.

Next, polyadenylated mRNA is separated from the extracted protein. Although several techniques have been developed to carry out this separation process, one preferred method is to chromatograph the polyadenylated mRNA on oligo (dT)-cellulose as described by Edmonds et al., 68 *Proc. Natl. Acad. Sci.*

1336 (1971); Aviv and Leder, 69 Proc. Natl. Acad. Sci. 1408 (1972); and, Maniatis et al., supra at 197. The oligo (dT)-cellulose column is prepared with a loading buffer and then the mRNA applied to the column. Thereafter, the column is initially washed with a buffer solution to remove the unpolyadenylated mRNA and then the polyadenylated mRNA is eluted from the column with a buffered, low ionic strength eluent. The integrity of the polyadenylated mRNA is verified by gel electrophoresis.

The polyadenylated mRNA is then sized by electrophoresis through methylmercury agarose. Gel fractions corresponding to different size classes of mRNA and then translated in vitro, by use of standard rabbit reticulocyte lysates technique, such as described by: Palmiter, 248 J. Biol. Chem. 2095 (1973); Pelham and Jackson, 67 Eur. J. Biochem. 246(1976); and, Lee et al., 253 J. Biol. Chem. 3494 (1978). Kits for the rabbit reticulocyte assay are commercially available from many sources, such as from Bethesda Research Laboratories, Gaithersburg, Maryland or New England Nuclear, Boston, Massachusetts. Alternatively, the mRNA translation can be carried out by microinjection of the mRNA into Xenopus laevis ("X. laevis") oocytes using standard techniques, such as described by Stoma et al., 79 Meth. Enzym. 68 (1981). Fluids liberated by either reticulocyte lysate translations, or by mRNA microinjected oocytes are then tested for the presence of IL-1 activity by use of the assay procedures set forth below. mRNA gel fractions which, when translated in vitro gave rise to IL-1 activity, are selected as a source of mRNA for cDNA construction.

In the X. laevis oocyte translation procedure, approximately 50 nanoliters ("nl") of mRNA (dissolved in sterile $H_2O$ at a concentration of 0.5-1 mg/ml) is injected into each oocyte. The oocytes are harvested from X. laevis (Nasco, Fort Atkinson, Wisconsin) and incubated in 150 ml of oocyte incubation medium (88 mM NaCl, 1 mM KCl, 2.4 mM $NaHCO_3$, 0.82 mM $MgSO_4 \cdot 7 H_2O$, 0.33 mM $Ca (NO_3)_2 \cdot 4H_2O$, 0.41mM $CaCl_2 \cdot 6H_2O$, 7.5 mM Tris base, 18 units/ml (11 μg/ml) penicillin G potassium, and 18 μg/ml streptomycin). The final pH of the medium is adjusted to 7.6 with HCl and then sterilized by filtration. After injection, the oocytes are placed in 0.1 ml of fresh oocyte incubation medium and incubated for 18 hours at 23°C in a 1.5 ml sterile conical polypropylene tube. After incubation, the oocyte supernates are removed and tested for biological activity as detailed below.

## Thymocyte Proliferation Assay

As noted above, mRNA translation by the rabbit reticulocyte lysates or the X. laevis oocytes is assayed by testing for the presence of IL-1 activity in the fluids liberated by the lysates or the oocyte extract. A first assay involves ascertaining the capacity of a sample of the lysate or oocyte extract to induce proliferation of thymocytes derived from CD-1 mice. In this assay, approximately 1 x $10^6$ thymocytes, obtained from 10 to 12 week old CD-1 mice (Charles River Breeding Laboratories, Wilmington, MA), are seeded in round bottom microplate wells (Corning Plastics, Corning, New York) in the presence of three-fold serial dilutions of the IL-1 containing fluid samples. The thymocytes are cultured in 150 microliters ("μl") of MEM containing 50 U/ml penicillin, 50 micrograms ("μg")/ml streptomycin, 2 millimolar ("mM") glutamine, 0.2 mM gentamycin, 10 mM HEPES buffer, ("Supplemented MEM"), pH 7.4, together with 3% v/v human serum and $10^{-5}$ M 2-mercaptoethanol. The samples are cultured for 72 hours at 37°C in an atmosphere of 5% $CO_2$ in air. Thereafter the cultures are pulsed for approximately 4 hours with 0.5 microcuries ("μCi") of tritiated thymidine ("$^3$H-Tdr"), (New England Nuclear, Boston, Massachusetts, 2 Ci/m M specific activity), after which the cultures are harvested onto glass fiber filter strips, for instance with the aid of a multiple-automated sample harvester. $^3$H-Tdr incorporation is then measured by liquid scintillation counting. Details of this procedure are set forth by Gillis et al., 120 J. Immunol. 2027 (1978) and in U.S. Patent 4,411,992.

By this thymocyte proliferation assay procedure, only the CD-1 thymocytes cultured in the presence of IL-1 incorporate $^3$H-Tdr in a dose dependent manner. CD-1 cells cultured in the absence of IL-1 incorporate only background levels of $^3$H-Tdr. IL-1 activity is calculated from the linear portion of the $^3$H-Tdr incorporation data in a manner similar to the procedure used by Gillis et al. supra, for determining interleukin-2 activity. Units of IL-1 activity are determined as the reciprocal dilution of a sample which generates 50% of maximal thymocyte $^3$H-Tdr incorporation as compared to a laboratory standard. For example, if a sample generates 50% of maximal thymocyte $^3$H-Tdr incorporation at a dilution of 1:15, then one unit ("U") of IL-1 is found in 1/15 of the 150 μl assay volume or 10 μl is said to contain one U of activity. The total sample would, therefore, contain 100 U [1,000 (μl/ml) ÷ 10 μl (per U)] of IL-1 activity/ml. See Gillis et al., supra.

## IL-1 Conversion Assay

A second alternative assay for IL-1 activity may be employed which takes advantage of the fact that IL-1 was found by applicants to convert an interleukin 2 ("IL-2") nonproducer murine tumor cell line, LBRM-33-145, to an IL-2 producer. In this assay LBRM-33-1A5 cells, ATCC No. CRL-8079, are inactivated by addition of 50 μg/ml of mitomycin C and incubated for 1 hour at 37° C. 100 μl of the inactivated LBRM-33-1A5 cells ($5 \times 10^5$ cells/ml) are cultured in 96-well flat-bottomed plates in the presence of an equal volume of the mitogen, phytohemagglutinin ("PHA") (1% final concentration) together with serial dilutions of IL-1 containing fluid samples. At hourly time intervals the existence of IL-2 activity, generated by IL-1 triggered, mitomycin C - inhibited LBRM-33-1A5 cells (and thus IL-1 activity), is directly ascertained by adding 50 μl of IL-2 dependent CTLL-2 cells ($8 \times 10^4$ cells/ml). The microwell cultures are then incubated for 20 additional hours followed by a 4 hour pulse with 0.5 μCi of $^3$H-Tdr (New England Nuclear, Boston, MA, 20 Ci/mM specific activity). Thereafter, the thymidine-pulsed cultures are harvested onto glass fiber filter strips with the aid of a multiple automated sample harvester (MASH II; Microbiological Associates, Bethesda, MD). $^3$H-Tdr incorporation is measured by liquid scintillation counting. Details of this procedure are set forth in Gillis et al. supra, and in U.S. Patent 4,411,992. In this assay, only the CTLL-2 cells cultured in the presence of IL-2 incorporate $^3$H-Tdr in a dose dependent manner. CTLL-2 cells cultured in the absence of IL-2 (and thus IL-1) incorporate only background levels of $^3$H-Tdr. This "conversion" assay has the advantage of being quicker (completion within 24 hours) and approximately 1000 to 10,000 times more sensitive than the above-discussed thymocyte proliferation assay. Nevertheless, both the "conversion" and "proliferation" assays may be employed in conjunction with the present invention.

Preparation of cDNA from mRNA

A library of double-stranded cDNA corresponding to the mRNA, as prepared and assayed above, is constructed by known techniques employing the enzyme reverse transcriptase. One such procedure which may be employed in conjunction with the present invention is detailed by Maniatis et al., supra at 230, as modified by Gubler and Hoffman, 25 Gene 263-269 (1983). Briefly, the polyadenylated mRNA is reverse transcribed by using oligo-dT, that has been hybridized to the polyadenylated tail of the mRNA, as a primer for a first cDNA strand. The second cDNA strand is synthesized using the enzymes DNA polymerase I, RNase H and E. coli DNA ligase. This procedure eliminates the S1 nuclease mediated cleaving of the hairpin loop formed in the 3' end of the initial cDNA strand, as would be required if standard cDNA synthesis techniques disclosed in Maniatus et al. were simply used. The double-stranded cDNA is fractionated by any convenient means to remove the shorter strands, thereby avoiding the needless cloning of small cDNA fractions.

It is to be understood that in accordance with the present invention, alternative standard procedures may be employed to prepare double-stranded cDNA from mRNA. One such alternative technique is disclosed by Land et al., 9 Nucl. Acids Res. 2251 (1981). In the Land et al. protocol, the hairpin loop also is not used as a primer for the second cDNA strand. Rather, the 3' end of the first cDNA strand is tailed with dCMP residues using terminal deoxynucleotidyl transferase ("TdT"). This produces a 3' tail of poly-C residues. Then the synthesis of the second strand is primed by oligdo-dG hybridized to the 3' tail. This technique is said to help avoid losing portions of the 5' tail of the second cDNA strand which might occur if the hairpin is cleaved with S1 nuclease, as in the Maniatis et al. protocol.

Cloning of cDNA

Next, the double-stranded cDNA is inserted within a cloning vector which is used to transform compatible prokaryotic or eukaryotic host cells for replication of the vector. Thereafter, the transformants are identified and plasmid DNA prepared therefrom.

To carry out the present invention, various cloning vectors may be utilized. Although the preference is for a plasmid, the vector may be a bacteriophage or a cosmid. If cloning occurs in mammalian cells, viruses also can be used as vectors.

If a plasmid is employed, it may be obtained from a natural source or artificially synthesized. The particular plasmid chosen should be compatible with the contemplated transformation host, whether a bacteria such as E. coli, yeast, or other unicellular microorganism. The plasmid should have the proper origin of replication for the particular host cell to be employed. Also, the plasmid should have a phenotypic property that will enable the transformed host cells to be readily identified and separated from cells that do not undergo transformation. Such phenotypic characteristics can include genes providing resistance to growth inhibiting substances, such as an antibiotic. Plasmids are commercially available that encode genes resistant to various antibiotics, including tetracycline, streptomycin, sulfa drugs, penicillin and ampicillin.

If E. coli is employed as the host cell, many possible cloning plasmids are commercially available which may be used in conjunction with the present invention. A preferred plasmid for performing the present invention is pBR322. This plasmid has been fully sequenced, as set forth in Sutcliffe, 43 Cold Spring Harbor Symp. Quant. Biol. 77 (1979). A significant advantage of this plasmid is that it has 11 known unique restriction sites, including the Pst I site in the ampicillin-resistant gene. This feature is particularly useful for cloning by the homopolymer tailing method.

If a bacteriophage is used instead of a plasmid, such phages should have substantially the same characteristics noted above for selection of plasmids. This includes the existence of a phenotypic marker and ligatable termini for attachment of foreign genes.

Preferably, in the present invention, the double-stranded cDNA, having blunt ends, is inserted into a plasmid vector by homopolymeric tailing. As is well known in the art, in this technique, complementary homopolymer tracks are added to the strands of the cDNA and to the plasmid DNA. The vector and double-stranded cDNA are then joined together by hydrogen bonding between complementary homopolymeric tails to form open, circular hybrid molecules capable of transforming host cells, such as E. coli.

In one procedure for homopolymeric tailing, approximately 50 to 150 dA nucleotide residues are added to the 3' ends of linearized plasmid DNA. A similar number of dT nucleotide residues are added to the 3' ends of the double-stranded cDNA and then the cDNA and plasmid joined together.

In an alternative and preferred method, dG tails are added to the 3' ends of the cloning vector that has been cleaved with an appropriate restriction enzyme. For instance, if the pBR322 plasmid is employed, the restriction enzyme Pst I may be used to digest the plasmid at the ampicillin resistant gene. Complementary dC tails are added to the 3' ends of the double-stranded cDNA prior to insertion of the cDNA segment in the plasmid with an appropriate annealing buffer.

It is to be understood that the double-stranded cDNA may be inserted within plasmid cloning vectors by other various standard methods. One such alternative technique involves attaching synthesized nucleotide linkers to the ends of the cDNA strands by using DNA ligase. The linkers are cleaved with a restriction enzyme to generate cohesive termini for insertion within a plasmid cleaved with the same restriction enzyme. Scheller et al., 196 Science 177-180 (1977); Maniatus et al., supra at 219.

The recombinant DNA plasmids, as prepared above, are used to transform host cells. Although the host may be any appropriate prokaryotic or eukaryotic cell, it is preferably a well-defined bacteria, such as E. coli or a yeast strain. Such hosts are readily transformed and capable of rapid growth in culture. Other forms of bacteria, such as salmonella or pneumococcus, may be substituted for E. coli. In place of bacteria, other unicellular microorganisms may be employed, for instance, fungi and algae. Whatever host is chosen, it should not contain a restriction enzyme that would cleave the recombinant plasmid.

If E. coli is employed as a host, preferable strains are MM294 and RR1. Protocols for transformation of the MM294 host by a plasmid vector are well known, as set forth in Maniatis et al., supra at 255; and, Hanahan, 166 J. Mol. Biol. 557 (1983). Protocols for transformation of the RR1 host by a plasmid vector are also well known as set forth in Bolivar et al., 2 Gene 95 (1977) and Peacock et al., 655 Biochem. Biophys. Acta. 243(1981). Other strains of E. coli which also could serve as suitable hosts include DH1 (ATCC No. 33849) and C600. These strains and the MM294 and RR1 strains are widely commercially available.

In transformation protocols, including those disclosed by Maniatis et al., supra, and Hanahan, supra, only a small portion of the host cells are actually transformed, due to limited plasmid uptake by the cells. The cells that have been transformed can be identified by placing the cell culture on agar plates containing suitable growth medium and a phenotypic identifier, such as an antibiotic. Only those cells that have the proper resistance gene (e.g., to the antibiotic) will survive. If the recombinant pBR322 plasmid is used to transform E. coli strain MM294, transformed cells can be identified by using tetracycline as the phenotypic identifier.

## Screening of cDNA Library by Hybridization Selection

The transformants, prepared above, are initially screened for the presence of the IL-1 gene by a hybrid selection method. In this procedure, individual transformants are chosen for screening and then pooled into groups. The groups are grown in liquid culture, and then the replicated plasmids are extracted from the transformants using any one of several well-known techniques, such as by alkaline lysis. Plasmid DNA is prepared by cleaving the plasmids at a unique restriction site(s) in the replicated plasmid. The resulting linearized DNA segments are filtered onto nitrocellulose. After baking of the plasmid DNA onto the nitrocellulose and removal of any loose DNA, the DNA that binds to the filter is hybridized with total (IL-1 active) mRNA, prepared above, in hybridization solution. Following hybridization, the unbound RNA is removed from the filter. Thereafter, the bound, hybrid selected mRNA is eluted and then translated in vitro by use of the rabbit reticulocyte lysate or frog X. laevis oocyte techniques detailed above. The fluids liberated by either reticulocyte lysate translation or by mRNA microinjected oocytes are then tested for the presence of IL-1 activity using the thymocyte proliferation and/or IL-1 conversion assays described above.

The particular pool(s) of clones that gave a positive signal are subdivided, and then the above hybridization selection procedure repeated to identify positive subpools. Next, positive subpools are divided into subsubpools or, if possible, into individual colonies and then the hybridization selection procedure again repeated. By this process, applicants have discovered one positive colony. Plasmid DNA, designated as 2C1, is prepared from the particular positive colony identified. The 2C1 clone is then characterized by nucleotide sequencing using the chain-termination method, discussed below under subheading "Characterization of Screened cDNA." The DNA sequence of the 2C1 clone is set forth in FIGURE 1 which illustrates the DNA composition of the IL-1 gene. The 2C1 clone, extending from nucleotide number 860 to nucleotide number 1,202 in FIGURE 1, composes a portion of the 3' flanking region of the IL-1 gene.

Screening of cDNA Library with Radiolabeled 2C1 Probe

The 2C1 clone is employed as a probe to rescreen the entire cDNA library prepared above in an effort to obtain a larger clone. The cDNA insert from the 2C1 clone is prepared by Pst I digestion of the plasmid and agarose gel electrophoresis. The 2C1 cDNA insert as thus prepared is radiolabeled prior to being used as a probe to screen the cDNA library. Various labeling techniques may be employed; however, because of its relatively large size, preferably the probe is labeled by "nick translation." In this well-known technique, as discussed by Rigby et al., 113 J. Molec. Bio. 237(1977), and Maniatis et al., supra at 108, nicks are introduced at widely separated sites in the DNA by very limited treatment with DNase I, thereby exposing a free 3'-OH group at each nick. DNA polymerase I is employed to incorporate an appropriate radiolabeled deoxynucleotide triphosphate, $^{32}$ P-DNTP, at the 3'-OH terminus and concurrently remove the nucleotide from the 5' side of the nick causing sequential movement of the nick along the DNA ("nick translation").

In the screening procedure, the transformants are pooled into groups each composed of approximately 2,000 transformants. The replicated plasmids are extracted by any one of several well-known techniques, such as alkaline lysis. DNA is prepared by cleaving the extracted plasmids at the Pst I restriction site. The resulting DNA segments are fractionated by electrophoresis on agarose gel and then analyzed by Southern blotting as described by Southern, 98 J. Mol. Biol. 503 (1975). The DNA that binds to the nitrocellulose filter in the Southern blotting procedure is hybridized with the labeled 2C1 probe. The specific DNA fragments that hybridize to this probe are identified by autoradiography.

The putative pool(s) of clones that discloses a strongly hybridizing band during autoradiography is plated out and used in direct bacterial colony hybridization on a nitrocellulose filter with the same labeled 2C1 cDNA probe. After completion of the hybridization, the nitrocellulose filter is monitored by autoradiography to identify a positive colony. By this procedure, applicants have discovered one such positive colony. Plasmid DNA, designated as 9H, is prepared from this particular colony.

The 9H clone is characterized by nucleotide sequencing using the chain-termination methods, described below. As illustrated in FIGURE 2, the nucleotide sequence of the 9H clone, which extends from nucleotide number 928 to nucleotide number 1,979 in FIGURE 1, comprises a rather large 3' flanking region of the IL-1 gene.

Screening of cDNA Library with Synthetic Oligonucleotide Probe

A radiolabeled synthetic oligonucleotide corresponding to a portion of the 2C1 clone is used as a probe to screen the above-prepared cDNA library. The hybridization of the synthetic oligonucleotide probe with plasmid cDNA prepared from the library clones is subsequently identified by autoradiography. Preferably, the probe is composed of the following composition from the antisense strand: 5'-GTTGGTTCCACTCTTAC-3'. This particular probe construct corresponds to nucleotide numbers 975 through 959 in FIGURE 1. This probe has the advantage of being short enough to be easily synthesized while being long enough to contain sufficient information to be useful as a probe for the IL-1 gene. Also, this same probe is employed as a primer in the chain-termination procedure, discussed below, for sequencing the 2C1 fragment.

Although the above-described oligonucleotide sequence is a preferred composition of the synthetic probe of the present invention, it is to be understood that probes of other compositions corresponding to other segments of the nucleotide sequence of the 2C1 fragment can be employed without departing from the spirit or scope of the present invention.

The synthetic oligonucleotide probe may be chemically synthesized by well-known techniques, such as by phosphodiester or triester methods. The details of the triester synthesis technique are set forth in Sood et al., 4 Nucl. Acid Res. 2557 (1977); and, Hirose et al., 28 Tec. Lett. 2449 (1978). After synthesis, the oligonucleotide probe is labeled with T4 polynucleotide kinase and $^{32}$ P-ATP. A standard protocol for the labeling procedure is set forth in Maniatis et al., supra at 122. Advantageously, the oligonucleotide probe can be synthesized with an OH-5' terminal thereby avoiding the phosphatase procedure typically required.

The labeled oligonucleotide probe is employed to screen Pvu II and Hind III digested plasmid DNA prepared as discussed above, from pools of approximately 2,000 transformants using the Southern blotting method described above. The specific DNA fragments that hybridize to the probe are identified by autoradiography. The particular pool(s) of clones that give a signal following autoradiography are plated out and used in direct bacterial colony hybridization on a nitrocellulose filter with the same above-identified oligonucleotide probe. After completion of the hybridization, the nitrocellulose filter is monitored by autoradiography to identify positive colonies. Applicants discovered two such positive colonies, from which plasmid DNA, designated as 5B and 8A, is prepared.

The nucleic acid sequence of both the 5B and 8A clones is analyzed by chain-termination method, as detailed below. As shown in FIGURE 1, the composition of the 5B clone extends from nucleotide number 591 to nucleotide number 995, and the composition of the 8A clone extends from nucleotide number 448 to 1,203. Also referring to FIGURE 2, both clones extend from within the open reading frame of the IL-1 gene to the 3' flanking region of the gene to overlap both the 2C1 and 9H clones previously isolated.

Screening of cDNA Library with 8A Clone-Derived Probe

A major 5' portion of the 8A clone was employed as a hybridization probe to further screen the cDNA library prepared above. Preferably, the probe is composed of the 5' Pst I-Hae III fragment from the 8A clone (corresponding to nucleotide numbers 448 through 771 in FIGURE 1). This probe is prepared by digesting the 8A clone with the restriction enzymes Hae III and Pst I and then isolating the desired fragment by electrophoresis. For use in the screening procedure, the 5' Pst I-Hae III fragment is radiolabeled by nick translation, as also discussed above.

The probe as thus prepared is employed to screen the cDNA library by use of the Southern blotting technique, discussed above. The specific DNA fragments that hybridize to the labeled probe are identified by autoradiography. By this process, applicants discovered one positive colony from which plasmid DNA, designated as 5F, was prepared. The nucleotide sequence of the 5F clone, as also determined by chain-termination method, is set forth in FIGURE 1 as nucleotide numbers 6 through 616. As shown in FIGURE 2, the 5F clone constitutes a major 5' portion of the open reading frame of the IL-1 gene, and overlaps the 5' portions of the 5B and 8A clones.

Screening of cDNA Library with 5F Clone-Derived Probe

The cDNA library prepared above was also screened with a probe composed of a 5' fragment of the 5F clone. This screening procedure was essentially the same as employed above with the 5' Pst I-Hae III probe. This particular probe, composed of the 5' Pst I-Hind III fragment, was prepared by digesting the 5' clone with Hind III and Pst I restriction enzymes and then employing electrophoresis to isolate the desired probe from the remaining plasmid fragments.

By use of this further screening procedure, applicants isolated two further clones designated as 18E and 10A. From nucleotide sequence analysis, applicants have ascertained that the 18E clone extends from nucleotide number -45 to nucleotide number 652 in FIGURE 1. The 10A clones extend from about nucleotide number -45 to about nucleotide number 1,150. As further illustrated in FIGURE 2, both clones overlap the 5' terminal portions of the 5B, 8A, 5F clones and extend into the 5' flanking region of the IL-1 gene. Also, the 10A clone spans the entire open reading frame of the gene. As will be appreciated, FIGURE 1 was prepared by assembling each of the above-identified clones, i.e., 2C1, 9H, 5B, 8A, 5F and 18E in correct order and orientation, which is made possible by the overlapping nature of these clones.

Characterization of Screened cDNA

The plasmid cDNA clones, isolated above, are sequenced using the chain-termination method. This method of nucleotide sequencing was originated by Sanger et al., 70 Proc. Natl. Acad. Sci. (USA) 5463-(1977). See U.S. Patent No. 4,322,499. Methods for chain-termination sequence determination are set forth in the Amersham Handbook entitled, M13 Cloning and Sequencing, Blenheim Cresent, London (1983) (hereinafter "Amersham Handbook"); Messing, 2 Recombinant DNA Technical Bulletin, NIH Publication No. 79-99,2, 43-48 (1979); Norrander et al., 26 Gene 101(1983); Cerretti et al., 11 Nucl. Acids Res. 2599 (1983); and, Biggin et al., 80 Proc. Natl. Acad. Sci. (USA) 3963(1983). M13 filamentous phage are employed as vectors to clone the DNA sequences of interest. These phage vectors provide single-stranded DNA templates which are readily sequenced by chain-termination method. This method involves priming a single-stranded template molecule with a short primer strand having a free 3' hydroxyl group and then using DNA polymerase to copy the template strand in a chain extension reaction using all four deoxyribonucleotide triphosphates, i.e., dATP, dCTP, dGTP, and dTTP (collectively referred to as "dNTPs"), with one of them being radiolabeled. In the synthesis reaction, a nucleotide specific, chain terminator lacking a 3'-hydroxyl terminus, for instance, a 2', 3' dideoxynucleotide triphosphate ("ddNTP"), is used to produce a series of different length chain extensions. The terminator has a normal 5' terminus so that it can be incorporated into a growing DNA chain, but lacks a 3' hydroxyl terminus. Once the terminator has been integrated into the DNA chain, no further deoxynucleotide triphosphates can be added so that growth of the chain stops. Four separate synthesizing reactions are carried out, each having a ddNTP of one of the four nucleotide dNPTs, i.e., dATP, dCPT, dGTP and dTTP. One of the normal dNTPs is radiolabeled so that the synthesized strands, after having been sorted by size on a polyacrylamide gel, can be autoradiographed. The chain extensions from the four reactions are placed side by side in separate gel lanes so that the pattern of the fragments from the autoradiography corresponds to the DNA sequence of the cloned DNA.

As discussed above, the DNA and corresponding amino acid sequences of the various clones, assembled together, is illustrated in FIGURE 1. The overlapping nature of the clones enable them to be arranged in the proper order and orientation. The nucleotide bases and amino acid residues are numbered beginning from the 5' end of the open reading frame for the IL-1 gene, i.e., the ATG codon/Met residue, marked with a star. The numbering of the amino acids extends to the Ala residue (No. 271) located adjacent the termination codon TAG. The numbering of the nucleotide bases extends to the end of the sequence shown in FIGURE 1. Also, the portion of the nucleotide sequence extending into the 5' flanking region of the IL-1 gene is designated with minus numbers. The coding region of mature IL-1 protein, as determined from the murine IL-1 gene identified in Lomedico et al., 312 Nature (London) 458 (November 29, 1984), extends from the Ser residue to the TAG termination codon (Nucleotides 337 to 813). Various restriction enzyme cleavage sites are also indicated in FIGURE 1.

In preparation for the sequencing procedures, the clones shown in FIGURE 2 are digested with various restriction endonucleases and then the resulting DNA fragments cloned into M13 phage vectors to form single stranded DNA templates. A universal primer is used to sequence the sense and antisense strands of the clones. Rather than relying on the sequencing results obtained from sequencing the entire length of the fragments with a single chain termination procedure, additional synthetically produced primers are used to initiate the chain termination procedure from other intermediate locations along the lengths of the strands. By this process, both strands of the various clones identified above are sequenced in overlapping fashion, thereby serving to redundantly confirm the sequences.

It is to be understood that rather than employing the chain-termination technique outlined above, other known methods may be utilized to sequence the IL-1 gene withcut departing from the spirit or scope of the present invention. For instance, the chemical degradation method of Maxam and Gilbert as set forth in 74 Proc. Nat'l Acad. Sci. (USA) 560(1977) can be used.

## Expression of Functional IL-1 from cDNA Clones

To determine whether the cDNA coding region of the IL-1 gene would encode functional human IL-1, the gene is expressed in mammalian cells and then tested with the above-described biological assays. A cDNA fragment containing the entire open reading frame of the IL-1 gene shown in FIGURE 1 is inserted into a plasmid vector derived in part from Simian virus 40 ("SV40"). The genome of this virus consists of a single, small covalently closed circular DNA molecule whose entire nucleotide sequence has been determined, Fiers et al., 237 Nature, (London) 113-120 (1978), and Reddy et al., 200 Science 494-502 (1978). The constructed expression vector, designated as pMLSV-IL-1$\alpha$ is illustrated in FIGURE 3.

The pMLSV-IL-1$\alpha$ expression vector is transfected into mammalian cells. After subsequent incubation, the cells are harvested and assayed for expression of mature human IL-1 by use of the direct thymocyte mitogenesis assay and IL-1 conversion assay discussed, supra. Applicants found that moderate levels of IL-1 was produced by cells transfected with the pMLSV-IL-1$\alpha$ plasmid with the bulk of the activity found in the cell extracts. This suggests that the mammalian cells were not capable of secreting IL-1 efficiently. This perhaps suggests that IL-1 is not actively secreted by macrophages and instead is released by damaged cells. Alternatively, perhaps macrophages may be able to export IL-1 by a mechanism that is distinct from that of other secretory proteins.

To investigate the essential portion of the IL-1 gene required for expression of biologically active IL-1, the C-terminal portion of the IL-1 gene extending from codon TCA (nucleotide numbers 337, 338 and 339) coding for $Ser^{113}$ and extending to codon GCG (nucleotide numbers 811, 812 and 813) coding for $Ala^{271}$

was inserted in an expression vector used to transform E. coli containing thermolabile repressor of $P_L$ transcription. Upon heat induction, applicants found that the cells produced very high levels of IL-1 activity. This establishes that IL-1 biological activity resides within the C-terminal 159 amino acids of the IL-1 molecule.

The processes and products of the present invention are further illustrated by the following examples.

## EXAMPLE 1

### Preparation of Polyadenylated mRNA

Leukocyte concentrates of a volume of 350-400 ml, obtained from human whole blood (mixture from Portland, Oregon Red Cross), were mixed with and diluted in $Ca^{++}$, $Mg^{++}$ free phosphate buffered saline ("PBS") layered onto Histopaque (Sigma Chemical Company, St. Louis, MO) and then centrifuged at 600 x g for 30 minutes at room temperature. The interface layer, consisting of the leukocytes, was recovered, washed with PBS and centrifuged at 400 x g for 10 minutes at room temperature. The cells were washed two more times in $Ca^{++}$, $Mg^{++}$ free PBS and centrifuged at 200 x g for 10 minutes after each washing.

Cells were then added to plastic culture flasks in Roswell Park Memorial Institute ("RPMI")-1640 medium together with 10% fetal bovine serum (v/v). Following a two-hour incubation at 37° C, nonadherent cells were decantled and the flasks were then replenished with additional serum supplemented RPMI-1640 medium containing 20 µg/ml E. coli LPS at 20 µg/ml. Sixteen hours later, adherent LPS stimulated cells were harvested for RNA.

Total RNA was extracted from the mononuclear cells generally by the method as described by Chirgwin et al., supra. In this procedure guanidinium thiocyanate was used to denature the cellular protein including the RNase at a rate that exceeds the rate of RNA hydrolysis by RNase. The mRNA was removed from the cellular protein by ethanol precipitation followed by resuspension (extraction) with 8 M guanidine HCl, 25 mM sodium acetate. Guanidine HCl extracted RNA was then reextracted with an equal volume of phenol/chloroform: isoamyl alcohol (25 volumes/24 volumes: 1 volume). The aqueous phase containing the RNA resulting from such extraction process was then rendered 50 mM sodium acetate, and precipitated by addition of 0.6 volume ethanol. RNA was collected by freezing at -20° C followed by centrifugation.

Thereafter, polyadenylated mRNA was separated from the extracted protein on an oligo (dT)-cellulose chromatography column using the method disclosed by Maniatis et al., supra at 197. Briefly, the column was prepared with application buffer composed of 20 mM Tris-Cl (pH 7.6), 0.5 M NaCl, 1 mM ethylene diamine tetraacetate ("ETDA") and 0.1% sodium dodecyl sulfate ("SDS"). The protein pellet was dissolved in water and application buffer and then loaded onto the column. The nonadsorbed material was eluted by initial washings with application buffer followed by additional washings with application buffer containing 0.1 M NaCl. The retained polyadenylated mRNA was eluted with buffers of reduced ionic strength composed of 10 mM Tris-Cl (pH 7.5), 1 mM EDTA and 0.05% SDS. The eluted polyadenylated mRNA was precipitated at -20° C with 1/10 volume sodium acetate (3M, pH 5.2) and 2.2 volumes of ethanol. After elution of the polyadenylated mRNA from the oligo (dT)-cellulose column, the integrity of the polyadenylated mRNA was confirmed by electrophoresis through agarose gels as detailed in Maniatis et al., supra at 199.

The polyadenylated mRNA was sized by electrophoresis through methylmercury agarose. Gel fractions corresponding to different size classes of mRNA were then translated in vitro, either by use of rabbit reticulocyte lysates or by injection in frog X. laevis oocytes as described above. Fluids liberated by either reticulocyte translations or by mRNA injected oocytes were then tested for the presence of IL-1 activity using the assays set forth above. mRNA gel fractions which, when translated in vitro gave rise to IL-1 activity, were selected as a source of mRNA for cDNA construction.

## EXAMPLE 2

### Construction of cDNA Library

A library of double-stranded cDNA corresponding to the mRNA was prepared from the purified mRNA in Example 1 by employing the standard procedure detailed by Maniatis et al., supra at 229 as modified by Gubler and Hoffman, supra. Oligo-dT was hybridized to the polyadenylated tail of the mRNA to serve as the primer for the reverse transcription of the first cDNA strand. The enzyme avian myeloblastosis virus ("AMV") reverse transcriptase synthesized the first cDNA strand by using the mRNA as a template. Briefly, the synthesis of the first cDNA strand is carried out in a reaction volume of 20-40 µl containing 50 mM Tris•HCl [pH 8.3], 10 mM $MgCl_2$, 10 mM dithiothreitol, ("DTT"), 4 mM Na • pyrophosphate, 1.25 mM dGTP, 1.25 mM dATP, 1.25 mM TTP, 0.5 mM dCTP, 15-20 µCi of [α -$^{32}$P] dCTP [3,000 Ci/mmol], 100 µg/ml of oligo ($dT_{12-18}$), 150 µg/ml mRNA (from Example 1), 3,000 units AMV reverse transcriptase/ml. The reaction was carried out at 43° C for thirty minutes and then stopped by adding EDTA to 20 mM. The reaction products were extracted with phenol and precipitated with ethanol out of 2 M $NH_4$ • acetate, as described by Okayama and Berg, 2 Mol. Cell. Biol. 161-170 (1982). The second cDNA strand was synthesized in a reaction containing 100 µl of 20 mM Tris•HCl as above [pH 7.5], 5 mM MgCl, 10 mM $(NH_4)_2SO_4$, 100 mM KCl, 0.15 mM β-NAD, 50 µg/ml BSA, 40 µm dNTPs, 8.5 units/ml of E. coli RNase H, 230 units/ml DNA polymerase I, 10 units/ml E. coli DNA ligase. This mixture is incubated at one hour for 12° C and then for a further hour at 22° C. Thereafter, EDTA is added to 20 mM to stop the reaction. The resulting double-stranded cDNA is extracted with phenol as described above.

The double-stranded cDNA was fractionated into size classes by Sephacryl S-400 (Pharmacia Fine Chemicals) column chromatography and monitored by analysis using alkaline agarose electrophoresis employing end-labeled fragments of pBR322 DNA as molecular-weight markers. DNA strands having a length of less than 500 bp were culled out to avoid needless cloning of these undersized cDNA fractions.

The double-stranded cDNA fractions, as prepared above, were inserted into the Pst I site of the pBR322 plasmid (Pharmacia Fine Chemicals) by the method disclosed by Maniatis et al., supra, beginning at 239. In this procedure the double-stranded cDNA was tailed with poly (dC) at its 3' ends. The plasmid pBR322 was digested with Pst I endonuclease and then tailed with poly (dG) at its 3' ends. The tailed plasmid DNA and the tailed cDNA were annealed with annealing buffer (0.1M NaCl, 10 mM Tris-Cl (pH 7.8) and 10 mM ETDA) to form novel recombinant plasmids. All restriction enzymes described herein are commercially available from New England Biolabs, Beverly, Massachusetts.

The recombinant plasmids were transformed into E. coli strain MM294 by using the procedure of Hanahan, supra in which the E. coli cells were prepared by growth in elevated levels of $Mg^{2+}$. The transformation hosts were plated and then transformants were identified by use of tetracycline as a phenotypic identifier. By use of this technique, applicants obtained approximately 4 x $10^4$ independent transform ants.

## EXAMPLE 3

Screening of cDNA Library by Hybridization Selection

The transformants, prepared in Example 2 above, were initially screened for the presence of the IL-1 gene by hybrid selection method. In this procedure, approximately 2,000 of the largest colonies of transformants were picked and grown in 96 well plates in liquid medium composed of Luria broth supplemented with tetracycline (25-75 µg/ml). Of these approximately 2,000 colonies, 16 pools of 48 each were chosen for screening. Plasmid DNA was removed from the chosen samples of host bacteria by standard alkaline lysis method detailed by Ish-Horowicz and Burke, 9 Nucl. Acid Res. 2989(1981) followed by gel filtration chromatography over a Sephacryl S-400 column (Pharmacia Fine Chemicals, Piscataway, New Jersey). The isolated plasmids were linearized by digestion to completion with Bam HI restriction enyzme. To this end, plasmids were redissolved in 50 µl of buffer (7 mM Tris, [pH 7.4], 7 mM magnesium chloride, 6 mM NaCl) and then 5 µl of Bam HI restriction endonuclease was added. This mixture was incubated at 37° C for one hour.

Next, 5 µg of plasmid DNA was prepared for nitrocellulose blotting according to the procedure of Panses et al., 78 Proc. Nat'l. Acad. Sci. (USA) 2253 (1981), by addition of 750 ul of 20 mM Tris-HCl, 1 mM EDTA [pH 7.5] followed by boiling for 10 min. Plasmid DNA was further denatured by addition of .750 µl of 1 normal NaOH for 20 minutes at room temperature. The mixture was then resuspended in 4.5 ml of buffer (250 mM Tris HCl [pH 8.0], 150 mM sodium citrate, 1.5 M NaCl, .25 M HCl).

Thereafter, the plasmid digests were filtered onto nitrocellulose with the aid of a Schleicher and Schuell manifold dot blot apparatus. After the transfer process, the filter was air-dried and baked for two hours at approximately 80° C to bind the DNA fragments to the nitrocellulose. Next, individual circular discs of the nitrocellulose (approximately 0.5 cm) corresponding to an individual clone pool were cut out from the nitrocellulose sheet and boiled for ten minutes in 2 ml of $H_2O$. The nitrocellulose spots were air-dried at 80° C for two hours.

The DNA that bound to the nitrocellulose disc was next hybridized with total IL-1 active mRNA that previously had been ethanol precipitated and resuspended in a hybridization solution. To this end, approximately 25 µg of total mRNA was suspended in 2.5 µl of $H_2O$, 5 µl of formamide and 2.5 µl of 4 x hybridization salts (400 mM PIPES buffer [pH 6.4], 1.6 mM NaCl and 40 mM EDTA [pH 8.0]). In the hybridization procedure, the circular nitrocellulose spots were placed into individual wells of a multiple well microtiter plate together with 10 μl of the above mRNA mixture and then covered with 100 μl of sterile paraffin oil. The microtiter plate was floated overnight on a water bath at 50° C. After overnight hybridization, the filters were removed and placed in individual polypropylene tubes and then washed ten times (one minute per wash) at 65° C with 1 x saline sodium citrate ("SSC") (20 x SSC is composed of 175.3 g of NaCl and 88.2 g of sodium citrate in 800 ml of $H_2O$, with pH adjusted to 7.0 with 10 N NaOH) and .2% sodium dodecyl sulfate ("SDS"). The filter was then washed five times with .2 x SSC.

Thereafter, the hybridized mRNA was eluted from the filter bound DNA by adding to the filter disc 200 μl of elution solution $H_2O$ with 6.6 μg/ml yeast tRNA (Sigma Chemical Co., St. Louis, Missouri), then incubating in a boiling water bath for three minutes and then cooling by immersion of the tube in ice. The solution was then transferred to a sterile 1.5 ml centrifuge tube. An additional 100 μl of elution solution was added to the filters and then the incubating and cooling procedure was repeated. This was pooled with the previous eluate. Next, NaCl is added to the eluted solution (300 μl total volume) to render it .3 M NaCl. Thereafter, two volumes of ethanol were added and the mixture was frozen at -70° C. The RNA was subsequently recovered by centrifugation.

The resulting ethanol precipitated, hybrid selected mRNA was translated in vitro by use of the rabbit reticulocyte lysate procedure, detailed above. The fluids liberated by the reticulocyte lysate translation were tested for the presence of IL-1 activity using the thymocyte proliferation and/or IL-1 conversion assays described above.

By the above procedure, one particular pool of clones gave a positive signal. This pool of 48 colonies was subdivided into eight pools of six colonies each and then the above-described hybridization selection procedure repeated. As a result, two positive subpools were identified, which subpools were divided into individual colonies (12 total) and the above hybridization procedure again repeated. By this process, applicants discovered one positive colony. Plasmid DNA, designated as 2C1, was prepared from this particular positive colony by standard alkaline lysis method, described supra.

**EXAMPLE 4**

Sequencing of Screened 2C1 Clone

The 2C1 clone was sequenced by the dideoxy chain-termination method essentially as described in the Amersham Handbook, supra, with the variations set forth below. The DNA segment was digested with Rsa I, Hae III and Pst I restriction endonucleases in various combinations and then the resulting DNA fragments were cloned into strains mp18 and mp19 of the M13 single-stranded filamentous phage vector (Amersham, Arlington Heights, Illinois). The mp18 and mp19 phage vectors, as set forth in Norrander et al. supra, contain the following unique cloning sites: Hind III; Sph I; Pst I; Sal I; Acc I; Hinc II; Xba I; BamHI; Xma I; Sma I; Kpn I; Sst I; and, EcoRI. The composition of the mp18 and mp19 vectors are identical, with the exception that the order of the above-identified restriction sites are reversed in the mp19 vector so that both strands of a DNA segment may be conveniently sequenced with the two vectors. The mp18 and mp19 vectors, with fragments of the cDNA segment of FIGURE 1 inserted therein, were used to transform E. coli JM103 and JM105 of the strain K12 (Bethesda Research Laboratories, Bethesda, Maryland) to produce replicate single-stranded DNA templates containing single-stranded inserts of the sense and antisense strands.

The synthetic universal primer: 5'-CCCAGTCACGACGTT-3' (P-L Biochemicals, Milwaukie, Wisconsin), was annealed to the single-strand DNA templates and used to prime DNA synthesis as described above. Thereafter, the extension fragments were size-separated by gel electrophoresis and autoradiographed from which the nucleotide sequences of the fragments were deduced. An additional primer was employed to prime synthesis from an intermediate location along the antisense strand of the DNA segment in FIGURE 2. A primer having the composition: 5'-GTTGGTTCCACTCTTAC-3', corresponding to nucleotide numbers 959 through 975 (FIGURE 1), was used to prime synthesis of the antisense strand. The composition of this primer strand was established from the sequencing information previously obtained by use of the universal primer.

By the above "walk down" method, both strands of the 2C1 clone were sequenced in an overlapping, redundant manner thereby confirming their nucleotide sequence. It is to be understood that other synthetic primers could have been employed to initiate chain extensions from other locations along the strands without departing from the scope of the present invention. The above primer strands were chemically synthesized by triester method as detailed by Sood et al., supra and Hirose et al., supra. It is to be understood, however, that other well-known techniques, such as by phosphodiester method, may be employed to synthesize the primer strands.

Deoxyadenosine 5' ($\alpha$ -[$^{35}$S] thio)triphosphate (hereinafter "dATP [$\alpha$ -$^{35}$S]") was used as the radioactive label in the dideoxy sequencing reactions. Also, rather than using the gel set forth at page 36 of the Amersham Handbook, a 6% polyacrylamide gel was employed (6% polyacrylmide gel, 0.4 mm thick, containing 7 M, urea 100 mM Tris borate (pH 8.1), and 2 mM EDTA).

As noted above, the nucleotide sequence of the 2C1 clone is illustrated in FIGURE 1 which includes the DNA composition of the IL-1 gene together with a large 3' flanking region of the gene as determined by combining together the individual clones isolated by the present invention. In FIGURE 1, the nucleotides are numbered from the beginning of the DNA sequence and the corresponding amino acids, as determined by the nucleotide sequence are set forth below the appropriate codons. The 2C1 clone extends from nucleotide number 860 to nucleotide number 1,202, and composes a portion of the 3' flanking region of the IL-1 gene.

## EXAMPLE 5

Screening of cDNA Library with Radiolabeled 2C1 Probe

The 2C1 clone was employed as a probe to rescreen the entire cDNA library in an effort to obtain a larger clone. The 2C1 probe was radiolabeled by nick translation by the procedure set forth in Maniatis et al., supra at 108, and as discussed above beginning at page 12. By this process the probe was labeled to a specific activity of approximately 3 x $10^8$ CPM/$\mu$g DNA. Prior to use in screening protocols, the labeled probe was denatured by boiling in water at 100° C for ten minutes followed by chilling on ice. In the screening procedure, the approximately 4 x $10^4$ transformants, as prepared above, were pooled into groups of 2,000 and then replicated plasmids extracted from the transformants by alkaline lysis, as discussed supra. Thereafter, DNA was prepared from the plasmids by digestion with Pst I and then the resulting DNA segments were fractionated by electrophoresis through 0.8% agarose gel with markers of appropriate size. The agarose gel was blotted onto a nitrocellulose filter using the standard method described by Southern, supra. After the transfer process, the filter was air-dried and baked for two hours at approximately 80° C under a vacuum to bind the DNA fragments to the nitrocellulose.

The bound DNA was thereafter hybridized with the labeled 2C1 probe. Briefly, the baked nitrocellulose was presoaked in 6 x SSC and then incubated at 68° C for 2-4 hours in prehybridization buffer composed of 6 x SSC, 0.5% NP40 detergent, 0.1% sarcosyl, 5 x Denhardt's solution (0.02% Ficoll, 0.02% polyvinyl pyrrolidone, 0.02% BSA) and 100 $\mu$g/ml denatured salmon sperm DNA (Sigma Type III, sodium salt). The filter was then incubated overnight at 68° C with the $^{32}$ P-labeled 2C1 probe (5 X $10^5$ cpm/ml) in hybridization solution. After overnight hybridization, the filter was washed extensively with 6 x SSC at room temperature and then for 30 minutes at 68° C with 0.6 x SSC. After air drying, the filter was subjected to autoradiography at -70° C.

From the radiography, applicants found several hybridizing bands. The pool of clones from which the plasmid DNA that produced the hybridizing bands was derived, was plated out and then used in direct bacterial colony hybridization on nitrocellulose paper with the labeled 2C1 probe under the same hybridization conditions as above. By this process, a single positive colony was identified. Plasmid DNA, designated as 9H, was prepared from this colony.

Thereafter, the nucleotide sequence of the 9H clone was ascertained by using the chain-termination method described in Example 4 above, with the variations set forth below. The 9H clone was prepared for sequencing by digesting with Rsa I, Hind III, Pst I and Sau 3A restriction endonucleases in various combinations prior to cloning the resulting DNA fragments into strains mp 18 and mp 19 of the M13 phage vector. In addition to synthesis with the universal primer described in Example 4 above, three additional primers were employed to prime synthesis from intermediate locations along the antisense and sense strands of the 9H clone. A primer having the composition: 5'-GTAATATGGGTAGAGTC-3', corresponding to nucleotide numbers 1,098 through 1,114, FIGURE 1, was used to prime synthesis of the antisense strand in the upstream direction from nucleotide number 1,097. The composition of this primer strand was established from the sequencing information previously obtained by using the universal primer. A second synthetic primer of the composition: 5'-TCATCTTGAGGCTCGGC-3', corresponding to nucleotide numbers 1,381 through 1,397, was used in sequencing the antisense strand in the upstream direction from nucleotide number 1,380. A third synthetic primer having the sequence: 5'-CATTTTGGTCCAAGTTG-3', corresponding to nucleotide numbers 1,581 through 1,598, was employed to sequence the sense strand in the downstream direction from nucleotide number 1,598.

As shown in FIGURE 1, the nucleotide in the sequence of the 9H clone as determined by the chain-termination method extends from nucleotide numbers 927 through 1,979 and comprises a rather large 3' flanking region of the IL-1 gene. Also referring to FIGURE 2, the 9H clone overlaps a major portion of the 2C1 clone, thereby confirming the nucleotide sequence of the IL-1 gene in this area.

## EXAMPLE 6

Screening of cDNA Library With 2C1 Clone-Derived Synthetic Oligonucleotide Probe

A radiolabeled synthetic oligonucleotide corresponding to a portion of the 2C1 clone was employed as a probe in screening the cDNA library prepared as set forth above in Example 3. The probe was composed of the following composition: 5'-GTTGGTTCCACTCTTAC-3'. This particular construct corresponds to the composition of the synthetic primer employed above in Example 4 in sequencing the 2C1 clone by chain-termination method. The oligonucleotide probe was chemically synthesized by a triester method as detailed by Sood et al., supra, and Hirose et al., supra.

After chemical synthesis has been completed, the 5' ends of the oligonucleotide probes were labeled with $^{32}P$. To facilitate labeling, the 5' ends of the oligonucleotide were synthesized with OH termini, thereby eliminating the phosphatase treatment which typically must be employed when labeling DNA fragments. The labeling protocol included adding 1 µl of synthetic oligonucleotides to 16 µl of $^{32}P$-ATP (7000 Ci/mMl), 1 µl (10 U) of T4 polynucleotide kinase and 2 µl of 10 X kinase buffer I. The 10 X kinase buffer I was composed of 0.5 M Tris-Cl (pH 7.6), 0.1 M $MgCl_2$, 50 mM dithiothreitol, 1 mM spermidine and 1 mM ETDA. The reaction was carried out at 37° C for 30 minutes, and thereafter the synthesized oligonucleotides were extracted with phenol/chloroform. The labeled probes were separated from unlabeled oligonucleotides by chromatography on or centrifugation through Sephadex G-50 columns (Pharmacia Fine Chemicals).

The labeled oligonucleotide probes were employed using the same Southern blotting method to screen linearized DNA prepared by digestion with Pvu II and Hind III and hybridization as detailed in Example 5 except that prehybridization was conducted at 45° C. Also, overnight probe incubation was conducted at 45° C followed by room temperature washing with 6 X SSC, and then a 5 minute wash with 0.6 X SSC. The particular pool(s) of clones that gave a positive signal following autoradiography were plated out and used in direct bacterial colony hybridization cn nitrocellulose paper with the same labeled oligonucleotide probe under the same hybridizing conditions as above. By this process, applicants discovered two positive colonies from which a plasmid DNA, designated as 5B and 8A, was prepared.

The 5B and 8A clones were analyzed by the dideoxy chain-termination method described above in Example 4, with the following variations. The 5B and 8A clones were digested with Rsa I and Pst I and Hae III restriction endonucleases and then the resulting DNA fragments were cloned into strains mp 18 and mp 19 of the M13 phage vector. In addition to the synthetic universal primer employed as described in Examples 3 and 5, two additional primers were employed to prime synthesis from immediate locations along the antisense and sense strands of the 8A clone. A primer having the composition: 5'-CAATTGTATGTGACTGC-3', corresponding to nucleotide numbers 568 through 584 (FIGURE 1), was used to prime synthesis of the sense strand in the downstream direction from nucleotide number 585. The composition of this primer strand was established from the sequencing information previously obtained by use of the universal primer. A second synthetic primer of the composition: 5'-TCAGCAGCACTGGTTGG-3' (corresponding to nucleotide numbers 597 through 613 in FIGURE 1) was used in sequencing the antisense strand in the upstream direction from nucleotide number 596. Through this sequencing procedure, applicants ascertained that the com-position of the 5B clone extends from nucleotide number 591 to nucleotide number 995, and the composition of the 8A clone extends from nucleotide number 448 to nucleotide number 1,203. Thus, as shown in FIGURE 2, the 8A clone overlaps both ends of the 5B clone. Also, both clones extend from within the open reading frame of the IL-1 gene to the 3' flanking region of the gene, and both clones overlap both the 2C1 and 9H clones, discussed above in Examples 3 and 5. The 8A clone in fact overlaps the entire length of the 2C1 clone, thereby redundantly confirming the nucleotide sequence of the IL-1 gene. The 8A clone, transformed into E. coli strain RR1, is on deposit with the American Type Culture Collection ("ATCC") 12361 Parklawn Drive, Rockville, Maryland 20852 under accession No. 39998.

## EXAMPLE 7

Screening of cDNA Library With 8A Clone-Derived Probe

The 5' Pst I-Hae III fragment from the 8A clone (corresponding to nucleotide numbers 448 through 771 in FIGURE 1), was employed as a hybridization probe to further screen the cDNA library, as prepared above in Example 3. This probe was prepared from the 8A clone by digestion with the restriction enzymes Pst I and Hae III followed by agarose gel electrophoresis. For use in the screening procedure, the 5' Pst I-Hae III fragment was radiolabeled by nick translation as discussed above in Example 3 to a specific activity of approximately 3 X $10^8$ CPM/μg DNA. Prior to use in the screening protocols, the labeled probe was denatured by boiling in $H_2O$ at 100 °C for ten minutes followed by chilling on ice. The probe as thus prepared was employed to screen the cDNA library in the manner described above in Example 5 by Southern blotting technique. By use of this procedure, one positive colony was identified from which plasmid DNA, designated as 5F, was prepared.

The nucleotide sequence of the 5F clone was ascertained by use of the dideoxy chain-termination method detailed above in Example 4, with the following variations. The 5F clone was digested with Pst I, Hind III and Eco RI, and restriction endonucleases in various combinations and then the resulting DNA fragments were cloned into mp 18 and mp 19 strains of the M13 phage vector.

By the above sequencing procedure, the 5F clone was found to be composed of the portion of the IL-1 clone shown in FIGURE 1 extending from the nucleotide number 6 through nucleotide number 616. Additionally referring to FIGURE 2, the 5F clone was found to constitute a major 5' portion of the open reading frame of the IL-1 gene. Also, this clone overlaps the 5' portions of the 5B and 8A clones discussed above in Example 6.

**EXAMPLE 8**

Screening of cDNA Library With 5F Clone-Derived Probe

In a manner similar to that described above in Example 7, the cDNA library was also screened with a probe composed of the 5' Pst I-Hind III fragment of the 5F clone. This particular probe, extending from nucleotide number 6 through nucleotide number 190 in FIGURE 1, was prepared by digesting the 5F clone with Pst I and Hind III restriction enzymes followed by agarose gel electrophoresis. The 5' Pst I-Hind III probe was employed to screen the cDNA library by use of the same Southern blotting technique, discussed above in Examples 5 and 7. By this further screening procedure, applicants discovered two positive colonies from which plasmid DNA, designated as 18E and 10A was prepared (deposited with the ATCC under Accession Nos. 39996 and 39997, respectively).

The 18E clone was also sequenced by dideoxy chain-termination method as described above in Example 4, with the following variations. The 18E clone was digested with Pst I, Eco RI and Hind III restriction endonucleases and then the resulting DNA fragments were cloned into strains mp 18 and mp 19 of the M13 phage vector.

By the above sequencing procedure, the 18E clone was found to be composed of the portion of the IL-1 clone shown in FIGURE 1 extending from nucleotide number -45 of the 5' flanking region of the IL-1 gene to nucleotide number 652. Also referring to FIGURE 2, this clone spans a major 5' portion of the open reading frame of the IL-1 gene, completely spans the 5F clone and overlaps the 5' portion of the 8A and 5B genes.

Similarly to the 18E clone, the 10A clone was found to begin in the 5' flanking region of the nucleotide gene, approximately nucleotide number -45. However, in the 3' direction, the 10A clone not only extends beyond the 3' end of the 18E clone, but also spans the entire length of the 5B clone and the open reading frame of the IL-1 gene, to about nucleotide number 1150.

Because of the overlapping nature of each of the above-discussed clones discovered by applicants, the entire open reading frame of the IL-1 gene, as well as the 5' flanking portion and an extensive 3' flanking portion of the gene was determined. The nucleotides in FIGURE 1 are numbered in each direction from the beginning of the open reading frame. The amino acids are numbered beginning from the open reading frame of the IL-1 gene, i.e., the Met residue, marked with a star, and extending to the termination codon TAG following residue No. 271. Based on the sequence of the murine IL-1 gene discussed in Lomedico et al., supra, applicants have determined that the coding region of the mature IL-1 protein extends from the Ser residue (nucleotide number 337) to the TAG termination codon (nucleotide number 813).

**EXAMPLE 9**

Exression of IL-1 Employing Entire Open Reading Frame of the IL-1 Gene

The entire open reading frame of the IL-1 gene shown in FIGURE 1 was inserted into a plasmid vector for a transfection of mammalian cells to ascertain whether functional human IL-1 could be expressed. The transfected cells and cell product was assayed for expression of IL-1 by their ability to promote thymocyte proliferation or induce IL-2 production in the IL-1 conversion assay. The 878 base pair Pst I-Hinc II fragment from the 10A clone, containing the entire open reading frame of the IL-1 gene, was inserted into the pMLSV-6 expression vector to produce a plasmid expression vector designated as pMLSV-IL-1α . The pMLSV-6 plasmid has been deposited with the ATCC under Accession No. 53081.

The pMLSV-6 vector was derived principally from SV40 whose genome consists of a single, small covalently closed DNA molecule that has been entirely sequenced, Fiers et al., supra, and Reddy et al., supra. The pMLSV-6 vector includes four primary parts, including the stippled box portion shown in FIGURE 3 which contains the control region of the SV40 plasmid (including the origin of DNA replication, enhancer elements and early and late promoters) (SV40 coordinates 5107-208). This vector portion was originally derived from the pSV2-dhfr vector as a Hind III-Pvu II fragment, Subramani et al., 1 Mol. Cell Biol. 854-864 (1981) and Lebowitz and Weissman, 87 Current Topics in Microbiology and Immunology 43 (1979). For use in the pMLSV-6 plasmid, the Pvu II site was converted into a BamHI site and the Hind III site converted to Xba I site.

Downstream from the early promoter, the pMLSV-6 vector includes a synthetic polylinker of the following composition:

5' CTAGATCGATGAATTCTCGAGATCTGCAGCTGGTACCAAGCTT 3'

'3 TAGCTACTTAAGAGCTCTAGACGTCGACCATGGTTCGAAGATC 5'

The polylinker has Xba I cohesive termini and contains the following restriction sites: Xba I; Cla I; EcoRI; Xho I; Bgl II; Pst I; Pvu II; Kpn I; and, Hind III.

The hatched box portion of the plasmid contains the SV40 small t antigen donor and acceptor splice junctions (SV40 coordinates 4035-4656) and the SV40 polyadenylation signal (SV40 coordinates 2469-2706), originally derived from the pSV2-dhfr plasmid as a Bgl II-BamHI fragment, Subramani, supra. The Bgl II site was converted to a Xba I site for correspondence with the adjacent terminal of the synthetic polymer.

The long thin line portion of the pMLSV-6 plasmid is derived from the plasmid pML2d, a derivative of plasmid pBR322, that lacks sequences inhibitory to DNA replication in mammalian cells, Server et al., 79 Proc. Natl. Acad. Sci. (USA) 7147-7151 (1982); and, Luskey and Botchan, 293 Nature 79-81 (1981).

The 10A clone was digested with Pst I and Hinc II restriction enzymes by standard techniques and inserted into the Pst I and Pvu II sites of the synthetic polylinker of the pMLSV-6 plasmid by standard techniques, for instance, as detailed in Maniatis et al., supra, to form plasmid vectors pMLSV-IL-1α.

The pMLSV-IL-1α plasmid as prepared above was transfected into COS-7 monkey kidney cells (ATCC, Rockville, MD) by standard techniques, for instance, by essentially using the procedures described by Luthman and Magnusson, 11 Nucl. Acid Res. 1295 (1983). Monolayers of COS-7 cells ($10^6$ cells per 10 cm plate) were washed twice with Dubecco's Modified Eagles Medium (DMEM) containing 10 mM Tris (pH 7.5) and exposed to 20 μg of pMLSV-IL-1α DNA per 5 ml of DMEM containing 10 mM Tris (pH 7.5), 250 μg/ml DEAE-Dextran (molecular weight $5 \times 10^5$; Sigma Chemical Company, St. Louis, MO.) and 100 μM chloroquine for 8 hours at room temperature. The cells were washed once more with DMEM containing 10 mM Tris (pH 7.5) and fed with growth medium (DMEM with 10% (v/v) fetal bovine serum). The cells were then incubated at 37° C for 5 days, after which time IL-1 activity was assayed in the media and in the cell extracts by use of both the IL-1 conversion assay and direct thymocyte mitogenesis assay. With the IL-1 conversion assay applicants found that the media exhibited an IL-1 activity of approximately 1,400 units, whereas the cell extract exhibited an activity of approximately 4,700 units.

**EXAMPLE 10**

Expression of Mature IL-1 with Portion of Human IL-1 Gene

A 3' portion of the IL-1 gene, extending from the codon coding for $Ser^{113}$ (nucleotide numbers 337, 338 and 339) to the codon coding for $Ala^{271}$ (nucleotide numbers 811, 812 and 813) was inserted into an expression vector to direct IL-1 expression in E. coli. This expression vector, designated as pILPα and as illustrated in FIGURE 4, was constructed from base plasmid pPLc28. The pPLc28 plasmid has been deposited with the ATCC under Accession No. 53082. The genome of plasmid pPLc28 contains the λ phage $P_L$ promoter. As illustrated in FIGURE 4, the pPLc28 plasmid contains an origin of replication (from plasmid pBR322) for high copy DNA replication in E. coli and an ampicillin resistant gene ("$Amp^r$", also from plasmid pBR322) for selection of transformed E. coli hosts.

The 3' portion of the IL-1 gene from the Alu I site (base number 351 in FIGURE 2) to the Nde I restriction site (base 850 in FIGURE 2) in the 3' flanking region of the gene was removed from the 10A clone by use of Alu I and Nde I restriction enzymes in a standard protocol, for instance as set forth in Maniatis et al., supra at 104. The IL-1 gene segment was cleaved from the 10A clone at the Alu I site, which is located 17 nucleotides downstream from the 5' terminal of the gene coding region (nucleotide number 337), because no convenient restriction site was found to correspond precisely at nucleotide number 337. A synthetic oligonucleotide was chemically synthesized to add back the 5' terminal portion of the coding region of the IL-l gene and also to create a ribosome binding site and a translation initiation codon at the 5' end of the coding region. The composition of the oligonucleotide, as shown in Table 1 below, includes an Eco RI cohesive 5' terminal, followed by ribosome binding site composed of the sequence: TAGGATAATTA and then by the initiation codon ATG. The initiation codon is followed by the 5' end of the coding region of the IL-1 gene terminating at the second nucleotide of $Ser^{117}$. Although the oligonucleotide shown in Table 1 was chemically synthesized by triester technique as detailed by Sood et al., supra and Hirose et al., supra, it is to be understood that the oligonucleotide can be prepared by other methods, such as by phosphodiester method.

**TABLE 1**

| | | | | | | | |
|---|---|---|---|---|---|---|---|
| | | | 113 | 114 | 115 | 116 | 117 |
| | | Met | Ser | Ala | Pro | Phe | Ser |
| 5' | AATTCTAGGATAATTA | ATG | TCA | GCA | CCT | TTT | AG |
| | GATCCTATTAAT | TAC | AGT | CGT | GGA | AAA | TC |

Also, rather than cleaving the coding region of the IL-1 gene at the Alu I site, the 10A clone in FIGURE 1 could be cleaved at a restriction enzyme site in the 5' flanking region of the coding portion of the gene. Thereafter, the nucleotides of the flanking portion can be sequentially removed by standard techniques. Next, a synthetic oligonucleotide composed of the Eco RI 5' cohesive terminal, ribosome binding site and translation initiation codon, could be linked to the 5' end of the IL-1 gene.

The pPLc28 expression vector was prepared for ligation to the synthetic oligonucleotide and the excised major portion of the coding region of the IL-1 gene by digestion of the vector to completion with restriction endonucleases Eco RI and Nde I by standard techniques, such as set forth in Maniatis et al., supra at 104. The desired larger fragment from the digestion of the pPLc28 plasmid was isolated by electrophoresis on 1.5% agarose gel at 100 volts at 25° C for 2-1/2 hours.

As shown in FIGURE 4, the synthetic DNA oligomer, the excised major portion of the coding region of the IL-1 gene and desired linearized pPLc28 fragment were ligated together in a reaction mixture composed of 50 nanograms (ng) of the pPLc28 vector fragment (Eco RI-Nde I), 50 ng of the major IL-1 cDNA fragment (Nde I-Alu I), 5 ng of synthetic oligonucleotide (Alu I-Eco RI), 1 unit of T4 DNA ligase and sufficient T4 ligase buffer (0.4M Tris (pH 7.4), 0.1M $MgCl_2$, 0.1M dithiothreitol, 10mM spermidine, 10mM ATP and 1 μg/ml BSA) to form a 20 μl reaction volume. The reaction was carried out by incubation at 25° C for 15 hours.

The resulting recombinant plasmid, designated as pILP α, was then transformed into E. coli strain ΔH1 which contains a defective λ lysogen encoding a thermolabile repressor of the $P_L$ promoter. In the transformation procedure, the entire ligation mix was added to competent ΔH1 E. coli (200 μl) (ATCC #33767). The mixture was allowed to set on ice for 30 minutes. The mixture then was pulsed with heat to 30° C for 4 minutes and grown for 2-1/2 hours in L-broth at 30° C. Transformed ΔH1 hosts were then plated in L-broth containing ampicillin, 70 μg/ml. For expression experimentation, ampicillin-resistant ΔH1 were grown in L-broth without ampicillin at 30° C to an absorbance at 720 nanometers of 0.5.

The cultures were then shifted to 43° C for 1 hour to promote derepression of the $P_L$ promoter. After 1 hour of culture, 1 ml samples of E. coli hosts were pelleted by centrifugation at 4° C and frozen by exposure to a mixture of dry ice and methanol. The 1 ml pellet was then resuspended in 50 μl of 7 M guanidine hydrochloride and refrozen on dry ice/methanol. The existence of biological activity in the guanidine extract was then ascertained by the thymocyte mitogenesis and IL-1 conversion assays supra. Applicants found that the pIL1p α plasmid expressed biological activity of over 838,433 units/ml of ΔH1 cells. This establishes that the coding region of the IL-1 gene resides in the 159 amino acid C-terminal portion of the IL-1 gene (nucleotide numbers 337 to 813).

As will be apparent to those skilled in the art in which the invention is addressed, the present invention may be embodied in forms other than those specifically disclosed above without departing from the invention. The particular embodiments of the present invention, described above, are therefore to be considered in all respects as illustrative and not restrictive.

**Claims**

**Claims for the following Contracting States: BE, CH, DE, FR, GB, IT, LI, LU, NL, SE**

**1.** Isolated DNA encoding mature human IL-1$\alpha$ wherein said human IL-1$\alpha$ has the amino acid sequence extending from residue no. 113 to residue no. 271 inclusive in Figures 1A and 1B.

**2.** DNA as claimed in claim 1 and having the nucleic acid sequence extending from nucleic acid base no. 337 to nucleic acid base no. 813 inclusive in Figures 1A and 1B.

**3.** A recombinant DNA cloning vector comprising DNA as claimed in claim 1 or 2.

**4.** A host transformed with a cloning vector as claimed in claim 3.

**5.** A recombinant DNA expression vector comprising DNA as claimed in claim 1 or 2 which is capable of inducing expression of the said DNA in a mammalian or bacterial host cell.

**6.** A mammalian or bacterial host cell transformed with an expression vector as claimed in claim 5.

**7.** A process for producing a transformant capable of expressing human IL-1$\alpha$ comprising transforming a host with a vector as claimed in claim 5.

**8.** A process for preparing human IL-1$\alpha$ comprising culturing a transformant produceable by a process as claimed in claim 7 so as to permit expression of the DNA encoding human IL-1$\alpha$.

**9.** Isolated mature human IL-1$\alpha$, wherein said human IL-1$\alpha$ has the amino acid sequence extending from residue no. 113 to residue no. 271 inclusive in Figures 1A and 1B.

**10.** Isolated mature human IL-1$\alpha$ as claimed in claim 9, for use as a medicament.

**11.** A pharmaceutical composition comprising a protein as claimed in claim 9 as an active ingredient.

**12.** A composition as claimed in claim 11 for use in the regulation of immunological responses.

**13.** A composition as claimed in claim 11 for use in the treatment of autoimmune disorders.

**14.** A composition as claimed in claim 11 for use in the treatment of arthritis, lupus erythematosis or wound or burn healing.

**15.** The use of a protein as claimed in claim 9 in the preparation of a medicament for use in
(i) the regulation of immunological responses;
(ii) the treatment of autoimmune disorders; or
(iii) the treatment of arthritis, lupus erythematosis or wound or burn healing.

**Claims for the following Contracting State: AT**

**1.** A process for preparing isolated DNA encoding mature human IL-1$\alpha$ wherein said human IL-1$\alpha$ has the amino acid sequence extending from residue no. 113 to residue no. 271 inclusive in Figures 1A and 1B, the process comprising coupling successive nucleotides together.

**2.** A process as claimed in claim 1 wherein the DNA has the nucleic acid sequence extending from nucleic acid base no. 337 to nucleic acid base no. 813 inclusive in Figures 1A and 1B.

3. A process for preparing a recombinant DNA cloning vector, the process comprising recombining DNA preparable by a process as claimed as claimed in claim 1 or 2 with cloning vector DNA.

4. A process for preparing a transformed host, the process comprising transforming a host with a cloning vector preparable by a process as claimed in claim 3.

5. A process for preparing a recombinant DNA expression vector which is capable of inducing expression of the human IL-1α DNA in a mammalian or bacterial host cell, the process comprising recombining DNA preparable by a process as claimed in claim 1 or 2 with expression vector DNA.

6. A process for producing a transformant capable of expressing human IL-1α comprising transforming a host with a vector producible by a process as claimed in claim 5.

7. A process for preparing human IL-1α comprising culturing a transformant produceable by a process as claimed in claim 6 so as to permit expression of the DNA encoding human IL-1α.

8. A process for preparing isolated mature human IL-1α, wherein said human IL-1α has the amino acid sequence extending from residue no. 113 to residue no. 271 inclusive in Figures 1A and 1B, the process comprising expressing DNA coding for the said human IL-1α.

9. A process for preparing a pharmaceutical composition, the process comprising admixing isolated mature human IL-1α producible by a process as claimed in claim 8.

10. A process as claimed in claim 9, wherein the composition is for use in the regulation of immunological responses.

11. A process as claimed in claim 9, wherein the composition is for use in the treatment of autoimmune disorders.

12. A process as claimed in claim 9, wherein the composition is for use in the treatment of arthritis, lupus erythematosis or wound or burn healing.

13. The use of a protein producible by a process as claimed in claim 9 in the preparation of a medicament for use in
    (i) the regulation of immunological responses;
    (ii) the treatment of autoimmune disorders; or
    (iii) the treatment of arthritis, lupus erythematosis or would or burn healing.

**Revendications**

**Revendications pour les Etats contractants suivants: BE, CH, DE, FR, GB, IT, LI, LU, NL, SE**

1. ADN isolé codant pour la IL-1α humaine mature, ladite IL-1α humaine possédant la séquence d'amino-acides s'étendant du résidu N° 113 au résidu N° 271 inclus sur les figures 1A et 1B.

2. ADN suivant la revendication 1 et comprenant la séquence d'acide nucléique s'étendant de la base d'acide nucléique N° 337 à la base d'acide nucléique N° 813 inclus sur les figures 1A et 1B.

3. Vecteur de clonage consistant en ADN recombinant, comprenant l'ADN suivant la revendication 1 ou 2.

4. Hôte transformé avec un vecteur de clonage suivant la revendication 3.

5. Vecteur d'expression d'ADN recombinant contenant l'ADN suivant la revendication 1 ou 2, qui est capable d'induire l'expression dudit ADN dans une cellule-hôte de mammifère ou de bactérie.

6. Cellule-hôte de mammifère ou de bactérie transformée avec un vecteur d'expression suivant la revendication 5.

7. Procédé de production d'un transformant capable d'exprimer la IL-1α humaine, consistant à transformer un hôte avec un vecteur suivant la revendication 5.

8. Procédé de préparation de IL-1$\alpha$ humaine, consistant à cultiver un transformant pouvant être produit par un procédé suivant la revendication 7 de manière à permettre l'expression de l'ADN codant pour la IL-1$\alpha$ humaine.

9. IL-1$\alpha$ humaine mature isolée, ladite IL-1$\alpha$ possédant la séquence d'amino-acides s'étendant du résidu N° 113 au résidu N° 271 inclus sur les figures 1A et 1B.

10. IL-1$\alpha$ humaine mature isolée suivant la revendication 9, destinée à être utilisée comme médicament.

11. Composition pharmaceutique comprenant une protéine suivant la revendication 9 comme ingrédient actif.

12. Composition suivant la revendication 11, destinée à être utilisée dans la régulation de réponses immunologiques.

13. Composition suivant la revendication 11, destinée à être utilisée dans le traitement de troubles auto-immuns.

14. Composition suivant la revendication 11, destinée à être utilisée dans le traitement de l'arthrite, du lupus érythémateux ou dans la cicatrisation de blessures ou de brûlures.

15. Utilisation d'une protéine suivant la revendication 9 dans la préparation d'un médicament destiné à être utilisé
(i) dans la régulation de réponses immunologiques ;
(ii) dans le traitement de troubles auto-immuns ; ou
(iii) dans le traitement de l'arthrite, du lupus érythémateux ou dans le cicatrisation de blessures ou de brûlures.

**Revendications pour l'Etat contractant suivant: AT**

1. Procédé de préparation d'ADN isolé codant pour le IL-1$\alpha$ humaine mature, dans lequel ladite IL-1$\alpha$ humaine possède la séquence d'amino-acides s'étendant du résidu N° 113 au résidu N° 271 inclus sur les figures 1A et 1B, procédé consistant à coupler ensemble des nucléotides successifs.

2. Procédé suivant la revendication 1, dans lequel l'ADN possède la séquence d'acide nucléique s'étendant de la base d'acide nucléique N° 337 à la base d'acide nucléique N° 813 inclus sur les figures 1A et 1B.

3. Procédé de préparation d'un vecteur de clonage d'ADN recombinant, consistant à recombiner l'ADN pouvant être préparé par un procédé suivant la revendication 1 ou 2 avec l'ADN du vecteur de clonage.

4. Procédé de préparation d'un hôte transformé, consistant à transformer un hôte avec un vecteur de clonage pouvant être préparé par un procédé suivant la revendication 3.

5. Procédé de préparation d'un vecteur d'expression d'ADN recombinant qui est capable d'induire l'expression de l'ADN de la IL-1$\alpha$ humaine chez une cellule hôte de mammifère ou de bactérie, consistant à recombiner l'ADN pouvant être préparé par un procédé suivant la revendication 1 ou 2 avec l'ADN du vecteur d'expression.

6. Procédé de production d'un transformant capable d'effectuer l'expression de la IL-1$\alpha$ humaine, consistant à transformer un hôte avec un vecteur pouvant être produit par un procédé suivant la revendication 5.

7. Procédé de préparation de IL-1$\alpha$ humaine, consistant à cultiver un transformant pouvant être produit par un procédé suivant la revendication 6 de manière à permettre l'expression de l'ADN codant pour la IL-1$\alpha$ humaine.

8. Procédé de préparation de IL-1α humaine mature isolée, dans lequel ladite IL-1α humaine possède la séquence d'amino-acides s'étendant du résidu N° 113 au résidu N° 271 inclus sur les figures 1A et 1B, consistant à provoquer l'expression de l'ADN codant pour ladite IL-1α humaine.

9. Procédé de préparation d'une composition pharmaceutique, consistant à incorporer la IL-1α humaine mature isolée pouvant être produite par un procédé suivant le revendication 8.

10. Procédé suivant la revendication 9, dans lequel la composition est destinée à être utilisée dans la régulation de réponses immunologiques.

11. Procédé suivant la revendication 9, dans lequel la composition est destinée à être utilisée dans le traitement de troubles auto-immuns.

12. Procédé suivant la revendication 9, dans lequel la composition est destinée à être utilisée dans le traitement de l'arthrite, du lupus érythémateux ou dans le cicatrisation de blessures ou de brûlures.

13. Utilisation d'une protéine pouvant être produite par un procédé suivant la revendication 9, dans la préparation d'un médicament destiné à être utilisé
(i) dans la régulation de réponses immunologiques ;
(ii) dans le traitement de troubles auto-immuns ; ou
(iii) dans le traitement de l'arthrite, du lupus érythémateux ou dans le cicatrisation de blessures ou de brûlures.

**Patentansprüche**
**Patentansprüche für folgende Vertragsstaaten: BE, CH, DE, FR, GB, IT, LI, LU, NL, SE**

1. Isolierte DNA, die für reifes menschliches IL-1α kodiert, wobei das menschliche IL-1α die Aminosäuresequenz aufweist, die von Rest Nr. 113 bis einschließlich Rest Nr. 271 in den Figuren 1A und 1B reicht.

2. DNA nach Anspruch 1 mit der Nukleinsäuresequenz, die von der Nukleinsäurebase Nr. 337 bis einschließlich der Nukleinsäurebase Nr. 813 in den Figuren 1A und 1B reicht.

3. Rekombinanter DNA-Klonierungsvektor, der DNA nach Anspruch 1 oder 2 umfaßt.

4. Wirt, der mit einem Klonierungsvektor nach Anspruch 3 transformiert ist.

5. Rekombinanter DNA-Expressionsvektor, umfassend DNA nach Anspruch 1 oder 2, der imstande ist, die Expression der DNA in einer Säuger- oder bakteriellen Wirtszelle zu induzieren.

6. Säuger- oder bakterielle Wirtszelle, transformiert mit einem Expressionsvektor nach Anspruch 5.

7. Verfahren zur Herstellung eines Transformanden, der imstande ist, menschliches IL-1α zu exprimieren, umfassend das Transformieren eines Wirts mit einem Vektor nach Anspruch 5.

8. Verfahren zur Herstellung von menschlichem IL-1α, umfassend das Kultivieren eines Transformanden, der durch ein Verfahren nach Anspruch 7 herstellbar ist, um so die Expression der für menschliches IL-1αkodierenden DNA zu erlauben.

9. Isoliertes, reifes menschliches IL-1α, worin das menschliche IL-1α die Aminosäuresequenz aufweist, die von Rest Nr. 113 bis einschließlich Rest 271 in den Figuren 1A und 1B reicht.

10. Isoliertes reifes menschliches IL-1α nach Anspruch 9 zur Verwendung als Medikament.

11. Pharmazeutische Zusammensetzung, umfassend ein Protein nach Anspruch 9 als aktiven Bestandteil.

12. Zusammensetzung nach Anspruch 11 zur Verwendung bei der Regulation von Immunreaktionen.

13. Zusammensetzung nach Anspruch 11 zur Verwendung bei der Behandlung von Autoimmunstörungen.

14. Zusammensetzung nach Anspruch 11 zur Verwendung bei der Behandlung von Arthritis, Lupus erythematosis oder der Wunden- oder Brandwundenheilung.

15. Verwendung eines Proteins nach Anspruch 9 bei der Herstellung eines Medikaments zur Verwendung bei
(i) der Regulation von Immunreaktionen;
(i) der Behandlung von Autoimmunstörungen; oder
(iii) der Behandlung von Arthritis, Lupus erythematosis oder Wunden- oder Brandwundenheilung.

**Patentansprüche für folgenden Vertragsstaat: AT**

1. Verfahren zur Herstellung von isolierter DNA, die für reifes menschliches IL-1$\alpha$ kodiert, worin das menschliche IL-1$\alpha$ die Aminosäuresequenz aufweist, die von Rest Nr. 113 bis einschließlich Rest 271 in den Figuren 1A und 1B reicht, wobei das Verfahren das Aneinanderkoppeln aufeinanderfolgender Nukleotide umfaßt.

2. Verfahren nach Anspruch 1, worin die DNA die Nukleinsäuresequenz aufweist, die von der Nukleinsäurebase Nr. 337 bis einschließlich der Nukleinsäurebase Nr. 813 in den Figuren 1A und 1B reicht.

3. Verfahren zur Herstellung eines rekombinanten DNA-Klonierungsvektors, wobei das Verfahren das Rekombinieren von DNA, die durch ein Verfahren nach Anspruch 1 oder 2 herstellbar ist, mit der DNA des Klonierungsvektors umfaßt.

4. Verfahren zur Herstellung eines transformierten Wirts, wobei das Verfahren das Transformieren eines Wirts mit einem Klonierungsvektor umfaßt, der durch ein Verfahren nach Anspruch 3 herstellbar ist.

5. Verfahren zur Herstellung eines rekombinanten DNA-Expressionsvektors, der imstande ist, die Expression der menschlichen IL-1$\alpha$-DNA in einer Säuger- oder bakteriellen Wirtszelle zu induzieren, wobei das Verfahren das Rekombinieren von DNA, die durch ein Verfahren nach Anspruch 1 oder 2 herstellbar ist, mit der DNA des Expressionsvektors umfaßt.

6. Verfahren zur Herstellung eines Transformanden, der imstande ist, menschliches IL-1$\alpha$ zu exprimieren, umfassend das Transformieren eines Wirts mit einem Vektor, der durch ein Verfahren nach Anspruch 5 herstellbar ist.

7. Verfahren zur Herstellung von menschlichem IL-1$\alpha$, umfassend das Kultivieren eines Transformanden, der durch ein Verfahren nach Anspruch 6 herstellbar ist, um so die Expression der für menschliches IL-1$\alpha$ kodierenden DNA zu erlauben.

8. Verfahren zur Herstellung von isoliertem reifem menschlichem IL-1$\alpha$, worin das menschliche IL-1$\alpha$ die Aminosäuresequenz aufweist, die von Rest Nr. 113 bis einschließlich Rest Nr. 271 in den Figuren 1A und 1B reicht, wobei das Verfahren die Expression von DNA umfaßt, die für das menschliche IL-1$\alpha$ kodiert.

9. Verfahren zur Herstellung einer pharmazeutischen Zusammensetzung, wobei das Verfahren die Zumischung von isoliertem reifem menschlichem IL-1$\alpha$ umfaßt, das durch ein Verfahren nach Anspruch 8 herstellbar ist.

10. Verfahren nach Anspruch 9, worin die Zusammensetzung für die Regulation von Immunreaktionen verwendbar ist.

11. Verfahren nach Anspruch 9, worin die Zusammensetzung für die Behandlung von Autoimmunstörungen verwendbar ist.

12. Verfahren nach Anspruch 9, worin die Zusammensetzung für die Behandlung von Arthritis, Lupus erythematosis oder Wunden- oder Brandwundenheilung verwendbar ist.

13. Verwendung eines Proteins, das durch ein Verfahren nach Anspruch 9 herstellbar ist, bei der Herstellung eines Medikaments zur Verwendung bei
(i) der Regulation von Immunreaktionen;
(ii) der Behandlung von Autoimmunstörungen; oder
(iii) der Behandlung von Arthritis, Lupus erythematosis oder Wunden- oder Brandwundenheilung.

# FIG.1A

-45 - TGA GGG AGT -36

0 *

CAT TTC ATT GGC GTT TGA GTC AGC AAA GAA GTC AAG ATC GCC 6
Met Ala 2

AAA GTT CCA GAC ATG TTT GAA GAC CTG AAG AAC TGT TAC AGT 48
Lys Val Pro Asp Met Phe Glu Asp Leu Lys Asn Cys Tyr Ser 16

GAA AAT GAA GAA GAC AGT TCC TCC ATT GAT CAT CTG TCT CTG 90
Glu Asn Glu Glu Asp Ser Ser Ser Ile Asp His Leu Ser Leu 30

AAT CAG AAA TCC TTC TAT CAT GTA AGC TAT GGC CCA CTC CAT 132
Asn Gln Lys Ser Phe Tyr His Val Ser Tyr Gly Pro Leu His 44

GAA GGC TGC ATG GAT CAA TCT GTG TCT GTG AGT ATC TCT GAA 174
Glu Gly Cys Met Asp Gln Ser Val Ser Leu Ser Ile Ser Glu 58

Hind III

ACC TCT AAA ACA TCC AAG CTT ACC GGA AAG GAG AGC ATG GTG 216
Thr Ser Lys Thr Ser Lys Leu Thr Gly Lys Glu Ser Met Val 72

GTA GTA GCA ACC AAC GGG AAG GTT CTG AAG AAG AGA CGG TTG 258
Val Val Ala Thr Asn Gly Lys Val Leu Lys Lys Arg Arg Leu 86

AGT TTA AGC CAA TCC ATC ACT GAT GAT GAC CTG GAG GCC ATC 300
Ser Leu Ser Gln Ser Ile Thr Asp Asp Asp Leu Glu Ala Ile 100

GCC AAT GAC TCA GAG GAA GAA ATC ATC AAG CCT AGG TCA GCA 342
Ala Asn Asp Ser Glu Glu Glu Ile Ile Lys Pro Arg Ser Ala 114

CCT TTT AGC TTC CTG AGC AAT GTG AAA TAC AAC TTT ATG AGG 384
Pro Phe Ser Phe Leu Ser Asn Val Lys Tyr Asn Phe Met Arg 128

Eco RI Aha II

ATC ATC AAA TAC GAA TTC ATC CTG AAT GAC GCC CTC AAT CAA 426
Ile Ile Lys Tyr Glu Phe Ile Leu Asn Asp Ala Leu Asn Gln 142

Rsa I

AGT ATA ATT CGA GCC AAT GAT CAG TAC CTC ACG GCT GCT GCA 468
Ser Ile Ile Arg Ala Asn Asp Gln Tyr Leu Thr Ala Ala Ala 156

TTA CAT AAT CTG GAT GAA GCA GTG AAA TTT GAC ATG GGT GCT 510
Leu His Asn Leu Asp Glu Ala Val Lys Phe Asp Met Gly Ala 170

TAT AAG TCA TCA AAG GAT GAT GCT AAA ATT ACC GTG ATT CTA 552
Tyr Lys Ser Ser Lys Asp Asp Ala Lys Ile Thr Val Ile Leu 184

# FIG.1B

```
AGA ATC TCA AAA ACT CAA TTG TAT GTG ACT GCC CAA GAT GAA   594
Arg Ile Ser Lys Thr Gln Leu Tyr Val Thr Ala Gln Asp Glu   198

GAC CAA CCA GTG CTG CTG AAG GAG ATG CCT GAG ATA CCC AAA   636
Asp Gln Pro Val Leu Leu Lys Glu Met Pro Glu Ile Pro Lys   212

ACC ATC ACA GGT AGT GAG ACC AAC CTC CTC TTC TTC TGG GAA   678
Thr Ile Thr Gly Ser Glu Thr Asn Leu Leu Phe Phe Trp Glu   226

ACT CAC GGC ACT AAG AAC TAT TTC ACA TCA GTT GCC CAT CCA   720
Thr His Gly Thr Lys Asn Tyr Phe Thr Ser Val Ala His Pro   240

AAC TTG TTT ATT GCC ACA AAG CAA GAC TAC TGG GTG TGC TTG   762
Asn Leu Phe Ile Ala Thr Lys Gln Asp Tyr Trp Val Cys Leu   254

GCA GGG GGG CCA CCC TCT ATC ACT GAC TTT CAG ATA CTG GAA   804
Ala Gly Gly Pro Pro Ser Ile Thr Asp Phe Gln Ile Leu Glu   268

AAC CAG GCG TAG GTC TGG AGT CTC ACT TGT CTC ACT TGT GCA   846
Asn Gln Ala End                                           271

    Hinc II
    |
GTG TTG ACA GTT CAT ATG TAC CAT GTA CAT GAA GAA GCT AAA   888

TCC TTT ACT GTT AGT CAT TTG CTG AGC ATG TAC TGA GCC TTG   930

TAA TTC TAA ATG AAT GTT TAC ACT CTT TGT AAG AGT GGA ACC   972

AAC ACT AAC ATA TAA TGT TGT TAT TTA AAG AAC ACC CTA TAT   1014

TTT GCA TAG TAC CAA TCA TTT TAA TTA TTA TTC TTC ATA ACA   1056

ATT TTA GGA GGA CCA GAG CTA CTG ACT ATG GCT ACC AAA AAG   1098

ACT CTA CCC ATA TTA CAG ATG GGC AAA TTA AGG CAT AAG AAA   1140

ACT AAG AAA TAT GCA CAA TAG CAG TTG AAA CAA GAA GCC ACA   1182

GAC CTA GGA TTT CAT GAT TTC ATT TCA ACT GTT TGC CTT CTG   1224

CTT TTA AGT TGC TGA TGA ACT CTT AAT CAA ATA GCA TAA GTT   1266
```

# FIG.1C

TCT GGG ACC TCA GTT TTA TCA TTT TCA AAA TGG AGG GAA TAA 1308

TAC CTA AGC CTT CCT GCC GCA ACA GTT TTT TAT GCT AAT CAG 1350

GGA GGT CAT TTT GGT AAA ATA CTT CTC GAA GCC GAG CCT CAA 1392

GAT GAA GGC AAA GCA CGA AAT GTT ATT TTT TAA TTA TTA TTT 1434

ATA TAT GTA TTT ATA AAT ATA TTT AAG ATA ATT ATA ATA TAC 1476

TAT ATT TAT GGG AAC CCC TTC ATC CTC TGA GTG TGA CCA GGC 1518

ATC CTC CAC AAT AGC AGA CAG TGT TTT CTG GGA TAA GTA AGT 1560

TTG ATT TCA TTA ATA CAG GGC ATT TTG GTC CAA GTT GTG CTT 1602

ATC CCA TAG CCA GGA AAC TCT GCA TTC TAG TAC TTG GGA GAC 1644

CTG TAA TCA TAT AAT AAA TGT ACA TTA ATT ACC TTG AGC CAG 1686

TAA TTG GTC CGA TCT TTG ACT CTT TTG CCA TTA AAC TTA CCT 1728

GGG CAT TCT TGT TTC ATT CAA TTC CAC CTG CAA TCA AGT CCT 1770

ACA AGC TAA AAT TAG ATG AAC TCA ACT TTG ACA ACC ATA GAC 1812

CAC TGT TAT CAA AAC TTT CTT TTC TGG AAT GTA ATC AAT GTT 1854

TCT TCT AGG TTC TAA AAA TTG TGA TCA GAC CAT AAT GTT ACA 1896

TTA TTA TCA ACA ATA GTG ATT GAT AGA GTG TTA TCA GTC ATA 1938

ACT AAA TAA AGC TTG CAA GAA AAA AAA AAA AAA AAA AAA AA 1979

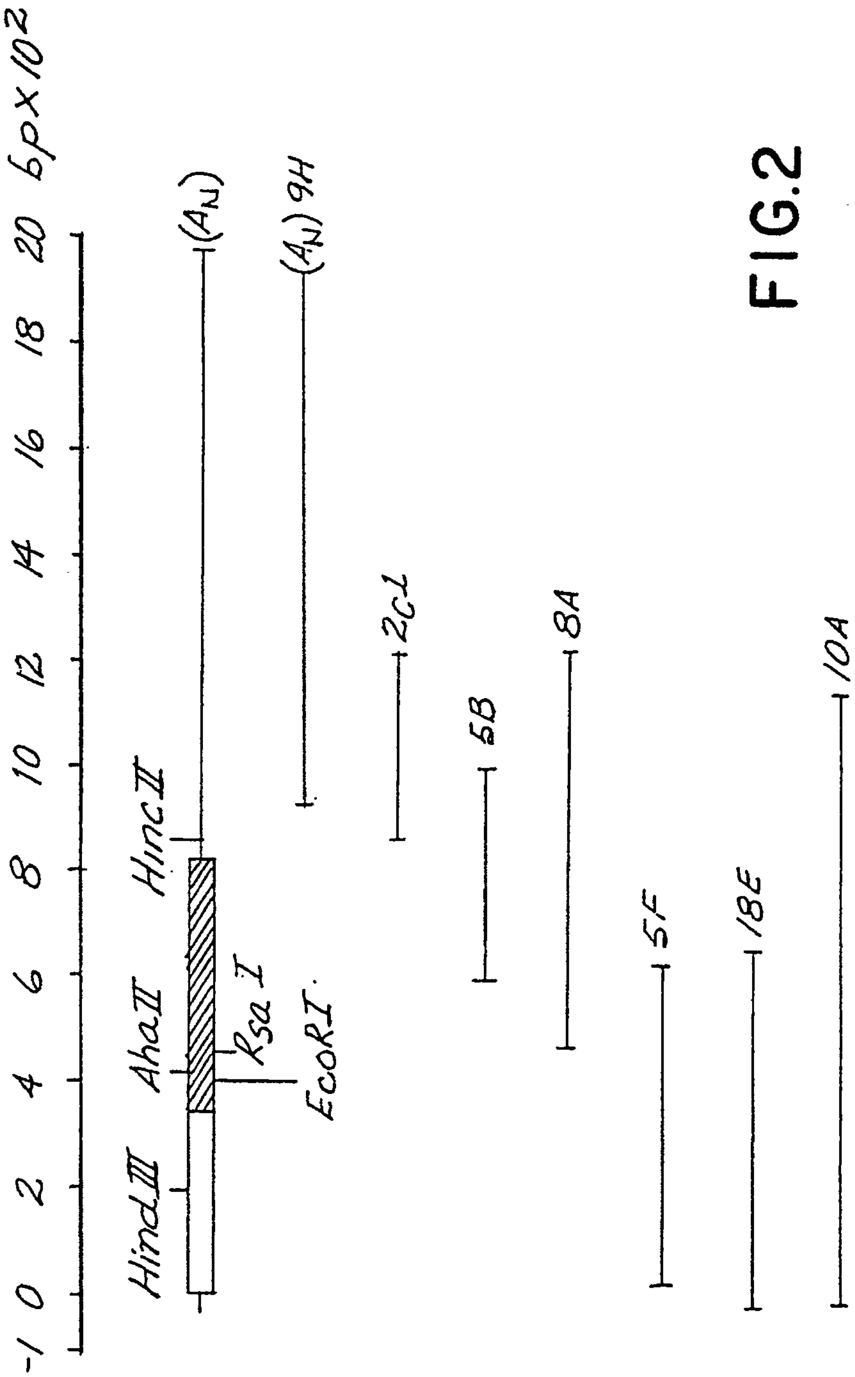

-1
0
2
4
6
8
10
12
14
16
18
20
bp x 10²
Hind III
Aha II
Hinc II
Rsa I
EcoRI
(AN)
(AN) 9H
2C1
5B
8A
5F
18E
10A


FIG.2

FIG.3

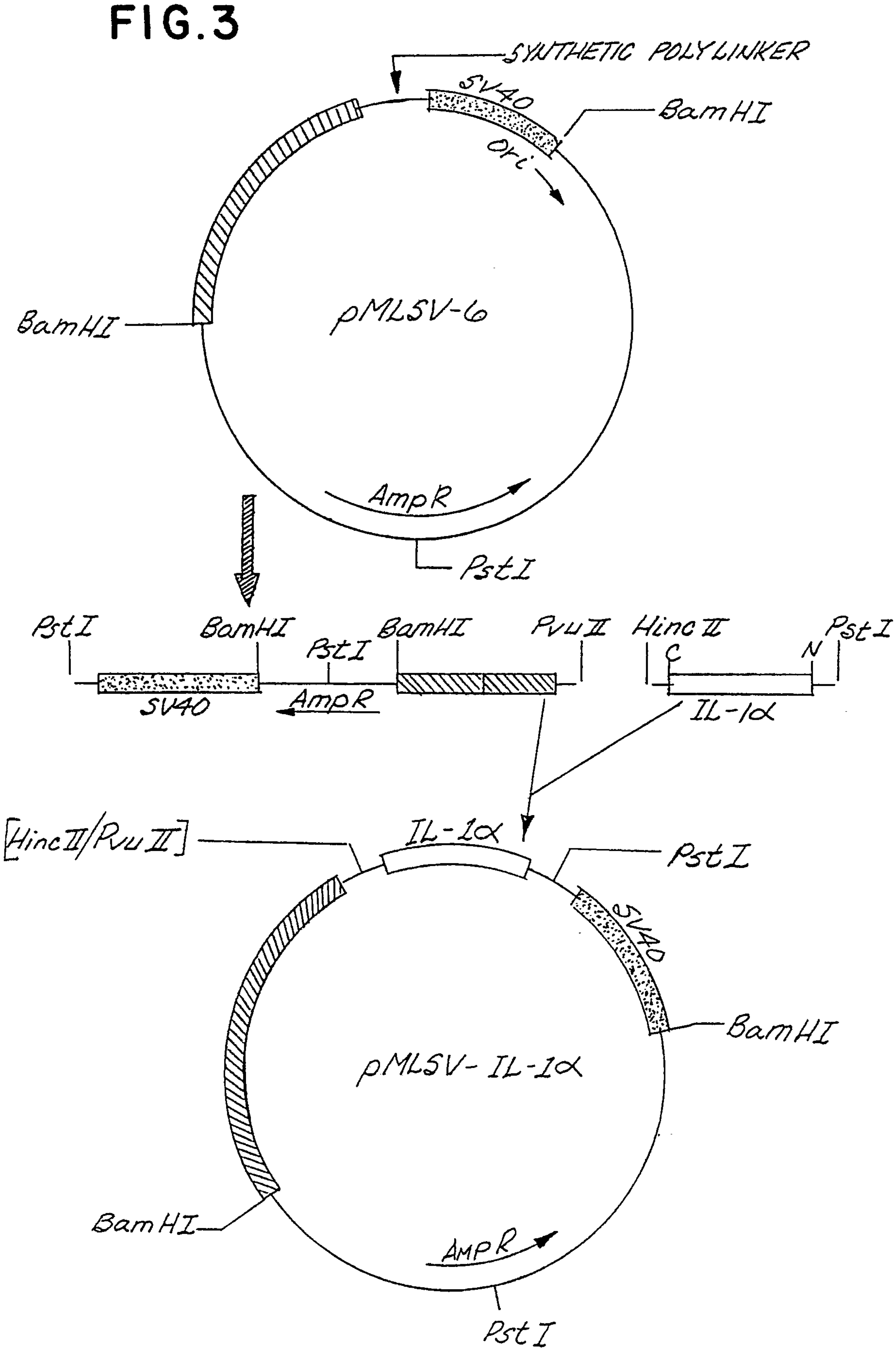
SYNTHETIC POLYLINKER
SV40
BamHI
Ori
BamHI
pMLSV-6
AmpR
PstI
PstI
BamHI
PstI
BamHI
PvuII
HincII
C
N
PstI
SV40
AmpR
IL-1α
[HincII/PvuII]
IL-1α
PstI
SV40
BamHI
pMLSV- IL-1α
BamHI
AmpR
PstI

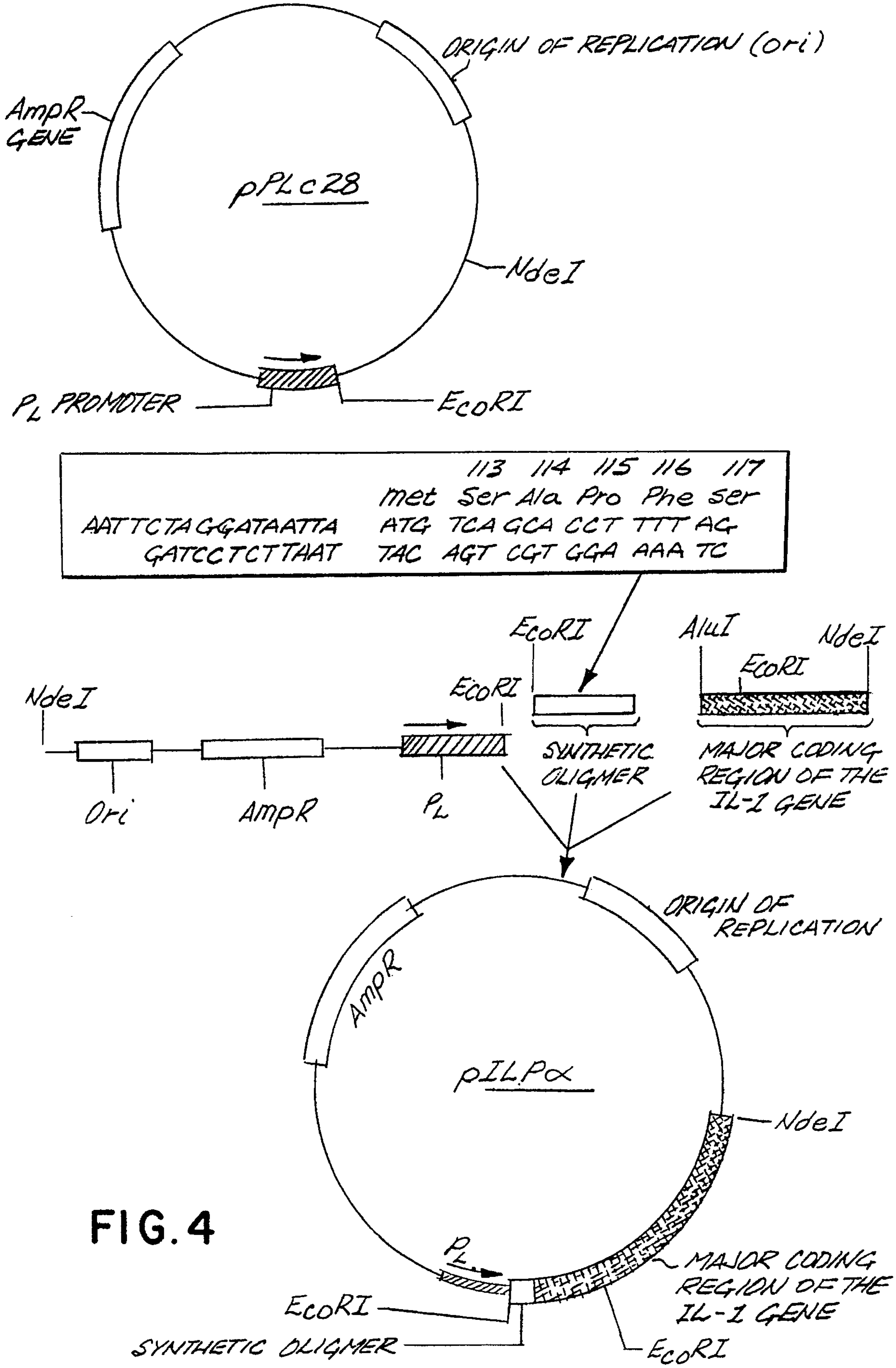
ORIGIN OF REPLICATION (ori)
AmpR GENE
pPLc28
NdeI
PL PROMOTER
EcoRI
113 114 115 116 117
met Ser Ala Pro Phe ser
AATTCTAGGATAATTA ATG TCA GCA CCT TTT AG
GATCCTCTTAAT TAC AGT CGT GGA AAA TC
EcoRI
AluI
NdeI
EcoRI
NdeI
EcoRI
Ori
AmpR
PL
SYNTHETIC OLIGMER
MAJOR CODING REGION OF THE IL-1 GENE
ORIGIN OF REPLICATION
AmpR
pILPα
NdeI
PL
MAJOR CODING REGION OF THE IL-1 GENE
EcoRI
SYNTHETIC OLIGMER
EcoRI

FIG. 4